# EUROPÄISCHE PATENTANMELDUNG

(11) **EP 4 480 463 A1**
(43) Veröffentlichungstag der Anmeldung: **25.12.2024**
(21) Anmeldenummer: 23180432.9
(22) Anmeldetag: 20.06.2023
(51) Int. Cl.: A61F 13/84

(54) **INKONTINENZARTIKEL MIT PH-REGULATIONSMITTEL**

(71) Anmelder: Paul Hartmann AG, 89522 Heidenheim (DE)
(72) Erfinder: DEBLER, Moritz, 89522 Heidenheim (DE); SHIMADA, Takahiro, Ibaraki-shi - Osaka, 567-0082 (JP)
(74) Vertreter: Paul Hartmann AG Patents & Licensing

(57) **Zusammenfassung**

Die vorliegende Erfindung betrifft einen Inkontinenzartikel für die Aufnahme von Körperausscheidungen umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper, wobei der Inkontinenzartikel ein zwischen dem Topsheet und dem Backsheet angeordnetes pH-Regulationsmittel aufweist, wobei das pH-Regulationsmittel in mindestens einem ersten Bereich und einem zweiten Bereich angeordnet ist, wobei der erste Bereich und der zweite Bereich jeweils eine Längserstreckung und eine Quererstreckung aufweisen, wobei der erste Bereich und der zweite Bereich in einer Querrichtung des Inkontinenzartikels voneinander beabstandet sind derart, dass ein zwischen dem ersten Bereich und dem zweiten Bereich verorteter und eine Längsmittelachse des Absorptionskörpers zumindest teilweise überfangender zentraler Bereich im Wesentlichen frei von pH-Regulationsmittel ist.

## Beschreibung

Die vorliegende Erfindung betrifft einen Inkontinenzartikel für die Aufnahme von Körperausscheidungen mit einem Mittel zur Regulation des pH-Wertes für die bevorzugte Verwendung durch Erwachsene.

Ein häufiges Problem bei der Verwendung von Inkontinenzartikeln ist das Auftreten von Hautreizungen oder Hautentzündungen, welche durch den Kontakt der Haut mit Körperausscheidungen wie beispielsweise Urin hervorgerufen werden. Oftmals kann sich eine sogenannte Inkontinenz-assoziierte Dermatitis (IAD) entwickeln, eine durch Kontakt mit einem Reizstoff wie Urin hervorgerufene Hautentzündung in der perinealen, perigenitalen oder periglutealen Region. Dabei spielen Veränderungen des pH-Wertes der Haut bei Kontakt mit dem alkalischeren Urin eine wesentliche Rolle. Um einen solchen Kontakt der Haut mit Urin zu verringern, weisen Inkontinenzartikel häufig superabsorbierende Materialien auf, welche große Mengen an Urin aufnehmen und dauerhaft binden können. Außerdem können bei einem entsprechenden Aufbau des Inkontinenzartikels, beispielsweise durch perforierte Deckschichten, Flüssigkeiten schneller in den Inkontinenzartikel aufgenommen und dabei von der Haut des Benutzers weggeleitet werden. Teilweise verfügen Inkontinenzartikel auch über Rillen oder Vertiefungen, welche zu einer besseren Flüssigkeitsleitung und -verteilung im Inkontinenzartikel beitragen sollen. Weiterhin werden manchmal Substanzen in das absorbierende Material des Saugkörpers eingebracht, um den pH-Wert der aufgenommenen Körperflüssigkeiten zu regulieren.

Jedoch kommt es häufig vor, dass bei einem Miktionsereignis eine große Menge an Urin in kurzer Zeit, also schwallartig, auf den Inkontinenzartikel trifft, so dass der Artikel diese Menge nicht schnell genug ableiten kann. Dabei kann sich der Urin zunächst in einem Schrittbereich des Inkontinenzartikels sammeln oder sich auf der Oberfläche des Saugkörpers vom Auftreffpunkt ausgehend in die Fläche ausbreiten, bevor er vom Saugkörper vollständig aufgesogen werden kann. Dadurch kommt es zu einem längeren und großflächigeren Hautkontakt, so dass leicht Hautreizungen entstehen können. Es besteht also weiterhin Bedarf an verbesserten Inkontinenzartikeln.

Inkontinenzartikel für Erwachsene sind seit langem bekannt und weisen häufig ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper auf. Diese können grundsätzlich auf vielfältige Weise ausgestaltet sein, beispielsweise als an den Körper anzulegende Inkontinenzprodukte wie Windeln oder als in die Kleidung einzulegende bindenartige Einlagen.

Im Stand der Technik finden sich Ansätze, Inkontinenzartikel mit einem Mittel zur pH-Wert-Kontrolle bereitzustellen. WO2012121932A1 lehrt einen absorbierenden Artikel mit in der Gebrauchssituation direktem Kontakt zur Haut des Nutzers. Der Artikel enthält eine Zusammensetzung umfassend Zitronensäure und ein Salz dieser Säure zur pH-Regulation von Urin, die besser wirke wenn sie keinen Kontakt zum Topsheet habe. Daher lehrt die WO2012121932A1, dass die Zusammensetzung mit absorbierendem Material vorgemischt sei oder zwischen beispielsweise zwei Saugkörperschichten oder einer Transferlage und dem Saugkörper anzuordnen sei. Dabei soll die Zusammensetzung im trockenen Zustand jedenfalls keinen Kontakt zum Topsheet haben.

Die Erfinder haben erkannt, dass eine solche Anordnung Nachteile mit sich bringen kann. Einerseits kann die Körperflüssigkeit, die erst im absorbierenden Kern oder nach dem Passieren einer Saugkörperlage in Kontakt mit der Zusammensetzung kommt, diese beim Vordringen in tiefere Saugkörperlagen leicht ausschwemmen oder überhaupt erst in tieferen Saugkörperlagen mit der Zusammensetzung in Kontakt kommen. Die Kontrolle des pH-Wertes an einer hautseitigen Oberfläche des Inkontinenzartikels kann dabei ungleichmäßig und/oder nur in ungenügendem Maße erfolgen. Andererseits kann bei einem Anfall einer größeren Menge an Körperflüssigkeit diese häufig nicht schnell genug ins Innere des Saugkörpers abgeleitet werden. Dabei kann - besonders bei einer konzentrierten Anordnung des pH-Regulationsmittels - sich die Körperflüssigkeit mit einer unerwünscht großen Menge des Mittels längere Zeit an der hautseitigen Oberfläche des Inkontinenzartikels halten. Dann besteht die Gefahr eines länger andauernden Kontakts mit der Haut des Nutzers und einer Hautirritation auch durch das der pH-Regulation dienende Mittel.

Der vorliegenden Erfindung liegt daher die Aufgabe zu Grunde, diese Nachteile zu überwinden.

Diese Aufgabe wird gelöst durch einen Inkontinenzartikel für die Aufnahme von Körperausscheidungen, umfassend eine Längsrichtung, eine Querrichtung, ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten und eine Längsmittelachse aufweisenden Absorptionskörper, wobei der Inkontinenzartikel ein zwischen dem Topsheet und dem Backsheet angeordnetes pH-Regulationsmittel aufweist, wobei das pH-Regulationsmittel in mindestens einem ersten Bereich und einem zweiten Bereich angeordnet ist, wobei der erste Bereich und der zweite Bereich jeweils eine Längserstreckung und eine Quererstreckung aufweisen, wobei der erste Bereich und der zweite Bereich in der Querrichtung des Inkontinenzartikels voneinander beabstandet sind derart, dass ein zwischen dem ersten Bereich und dem zweiten Bereich verorteter und die Längsmittelachse des Absorptionskörpers zumindest teilweise überfangender zentraler Bereich im Wesentlichen frei von pH-Regulationsmittel ist.

Durch die menschliche Anatomie bedingt stehen Inkontinenzartikel häufig vor allem in ihren seitlichen Abschnitten in näherem Kontakt mit der Haut des Nutzers. Dies trifft in besonderem Maße auch in einem Schrittabschnitt des Inkontinenzartikels zu, der zwischen den Beinen eines Nutzers, also im Bereich der Interlabialfalte und/oder der Glutealfalte zu liegen kommt. Gerade in seitlichen Bereichen des Inkontinenzartikels ist ein näherer Kontakt zur Verbesserung der Auslaufsicherheit erwünscht, so dass Inkontinenzartikel oftmals so ausgebildet sind, dass sie an der Haut des Nutzers direkt anliegen können. Außerdem kann es auch zu einer gewissen seitlich durch die Beine ausgeübten Kompression des Inkontinenzartikels kommen. Auf vorteilhafte Weise kann durch die erfindungsgemäße Anordnung das pH-Regulationsmittels vor allem dort wirken, wo der Inkontinenzartikel näher an die Haut gelangen kann. In dem zentralen Bereich hingegen, der im Wesentlichen frei von pH-Regulationsmittel ist, kann ein Kontakt zu höheren Mengen an pH-Regulationsmittel vermieden werden. Dadurch kann eine Gefahr von Irritationen der typischerweise mittig zu liegen kommenden, häufig empfindlicheren perigenitalen und/oder periglutealen Hautareale verringert werden. Das pH-Regulationsmittel kann also vorrangig dort wirken, wo eine pH-Kontrolle aufgrund der Nähe zur Haut wünschenswert ist, wobei die Gefahr von Irritationen empfindlicherer Hautareale verringert werden kann.

Der Begriff "Längsrichtung" ist im Rahmen dieser Erfindung die Richtung von einem in der Gebrauchssituation bauchseitig zu liegen kommenden, also vorderen Ende zu einem rückenseitig zu liegen kommenden, also hinteren Ende des Inkontinenzartikels. Die "Querrichtung" ist zu verstehen als rechtwinklig zur Längsrichtung in der flächenhaften Erstreckung des gegebenenfalls aufgefalteten und jedenfalls flach ausgebreiteten Inkontinenzartikels orientierten Richtung des Inkontinenzartikels.

Topsheet- und Backsheetmaterialien sind im Fachgebiet hinreichend bekannt. Das Backsheet kann insbesondere ein atmungsaktives, jedoch im Wesentlichen flüssigkeitsdichtes Folienmaterial umfassen oder daraus gebildet sein. Bevorzugt ist das Backsheet aus einem Vlies-Folien-Laminat ausgebildet. Ein Vlies-Folien-Laminat kann für den Nutzer vorteilhafte haptische und/oder akustische Eigenschaften aufweisen, da das Material sich häufig weicher anfühlt und weniger raschelt als ein Folienmaterial ohne Vliesanteil. Das Topsheet ist zumindest bereichsweise, insbesondere vollständig flüssigkeitsdurchlässig ausgebildet. Das Topsheet umfasst oder ist gebildet aus vorzugsweise einem Filmmaterial und/oder einem Vliesmaterial. Bevorzugt ist das Topsheet ein Spunbond-Vliesmaterial umfassend oder bestehend aus künstlichem Polymer wie beispielsweise Polyolefin, insbesondere Polypropylen. Auch Vliesmaterialien umfassend oder bestehend aus natürlichen Fasern sind grundsätzlich denkbar.

In einer bevorzugten Ausführungsform ist der erste Bereich und der zweite Bereich jeweils streifenförmig ausgebildet, derart dass die jeweilige Längserstreckung des ersten Bereichs und des zweiten Bereichs größer ist als die Quererstreckung des jeweiligen ersten Bereichs oder zweiten Bereichs. Dadurch kann auf vorteilhafte Weise die in Längsrichtung des Inkontinenzartikels weitergeleitete Körperflüssigkeit auch in von einem Bereich des ersten Auftreffens weiter entfernten Regionen des Inkontinenzartikels in ihrem pH-Wert effektiv reguliert werden. Die Längserstreckung eines jeweiligen ersten Bereichs oder zweiten Bereichs ist zu messen entlang der Längsrichtung des Inkontinenzartikels. Die Quererstreckung des jeweiligen ersten Bereichs oder zweiten Bereichs ist zu messen entlang der Querrichtung des Inkontinenzartikels. Die Längserstreckung und die Quererstreckung ist jeweils eine maximale Erstreckung des jeweiligen Bereichs in der jeweiligen Richtung des Inkontinenzartikels.

Der Begriff "streifenförmig" ist so zu verstehen, dass der erste Bereich und/oder der zweite Bereich bevorzugt im Wesentlichen rechteckig ist. Der erste Bereich und/oder der zweite Bereich kann jedoch auch oval, ellipsenförmig, mandelförmig, kurvenförmig, wellenförmig, L-förmig, C-förmig, knochenförmig oder in jeder anderen denkbaren Form ausgebildet sein, sofern der jeweilige erste Bereich und/oder der zweite Bereich in der Längsrichtung weiter erstreckt ist als in der Querrichtung. Maßgeblich hierbei ist der Abstand in Querrichtung von einem der Längsmittelachse des Absorptionskörpers nächstgelegenen Ende zu einem der Längsmittelachse des Absorptionskörpers entferntesten Ende des jeweiligen ersten Bereichs oder zweiten Bereichs.

In einem alternativen Ausführungsbeispiel ist das pH-Regulationsmittel in mehr als zwei Bereichen, also in einem oder mehreren zu dem ersten Bereich und dem zweiten Bereich zusätzlichen Bereich oder Bereichen, beispielsweise in dem ersten Bereich und dem zweiten Bereich und einem dritten Bereich und/oder einem vierten Bereich und/oder einem fünften Bereich und/oder einem sechsten Bereich und/oder einem siebten Bereich und/oder einem achten Bereich angeordnet. Der zusätzliche Bereich oder die zusätzlichen Bereiche ist oder sind jeweils von dem ersten Bereich und/oder von dem zweiten Bereich und/oder voneinander in Querrichtung beabstandet derart, dass zwischen jeweils zwei voneinander beabstandeten und einander nächstgelegenen Bereichen aus der Gruppe erster Bereich, zweiter Bereich, dritter Bereich, vierter Bereich, fünfter Bereich, sechster Bereich, siebter Bereich, achter Bereich, jeweils ein im Wesentlichen pH-Regulationsmittel-freier weiterer Bereich angeordnet ist.

Der zusätzliche Bereich oder die zusätzlichen Bereiche ist oder sind vorzugsweise streifenförmig und bezüglich einer jeweiligen Längserstreckung bevorzugt analog zu dem ersten Bereich und/oder dem zweiten Bereich ausgebildet.

Bevorzugt sind der erste Bereich und der zweite Bereich spiegelsymmetrisch beidseits der Längsmittelachse des Absorptionskörpers ausgebildet. Hierbei bietet sich für den Nutzer der Vorteil, dass das pH-Regulationsmittel beidseits der Längsmittelachse des Absorptionskörpers vorhanden, insbesondere über eine im Wesentlichen gleich große und gleichförmige Fläche hinweg vorhanden ist. Die Gefahr einer stark unterschiedlichen pH-Regulationsleistung zwischen dem ersten Bereich und dem zweiten Bereich und damit einseitiger erhöhter Gefahr von Hautirritationen kann somit herabgesetzt sein.

In einem bevorzugten Ausführungsbeispiel weist die jeweilige Quererstreckung des ersten Bereichs und/oder des zweiten Bereichs einen Wert von jeweils 10-60 mm, insbesondere 15-50 mm, weiter insbesondere 20-40 mm auf. Auf vorteilhafte Weise kann daher eine pH-Regulation über eine relativ große Gesamterstreckung in Querrichtung hinweg erfolgen. Insbesondere bei höherem Flüssigkeitsanfall, bei dem es leicht zu einer eher großflächigen Verteilung der Flüssigkeit an der hautseitigen Oberfläche des Inkontinenzartikels kommen kann, ist es daher möglich, über einen größeren Bereich des Inkontinenzartikels hinweg und insbesondere zu seitlichen Rändern des Artikels hin den pH-Wert rasch zu regulieren.

In einer bevorzugten Ausführungsform grenzt der zentrale Bereich direkt an den ersten Bereich und den zweiten Bereich an. Dadurch wird auf vorteilhafte Weise eine pH-Regulation beidseits des zentralen Bereichs, mithin beidseits der Längsmittelachse des Absorptionskörpers ermöglicht.

Eine Quererstreckung des zentralen Bereichs weist vorzugsweise einen Wert von 3-50 mm, insbesondere 5-40 mm, weiter insbesondere 8-35 mm, weiter insbesondere 10-30 mm, weiter insbesondere 15-25 mm auf. Die Quererstreckung des zentralen Bereichs ist hierbei zu verstehen als eine minimale Erstreckung des zentralen Bereichs in der Querrichtung des Inkontinenzartikels, mithin ein Abstand in Querrichtung von einem der Längsmittelachse des Absorptionskörpers nächstgelegenen Ende des ersten Bereichs zu einem der Längsmittelachse des Absorptionskörpers nächstgelegenen Endes des zweiten Bereichs.

Bevorzugt beträgt ein Verhältnis der jeweiligen Quererstreckung des ersten Bereichs und/oder des zweiten Bereichs zu der Quererstreckung des zentralen Bereichs jeweils 0,2-20, insbesondere 0,7-15, weiter insbesondere 1-10, weiter insbesondere 2-5.

In einer bevorzugten Ausführungsform weist die jeweilige Längserstreckung des ersten Bereichs und/oder des zweiten Bereichs einen Wert von mindestens 50 mm, insbesondere mindestens 60 mm, weiter insbesondere mindestens 70 mm, weiter insbesondere mindestens 80 mm, weiter insbesondere mindestens 90 mm, weiter insbesondere mindestens 100 mm auf. Hierdurch wird auf vorteilhafte Weise ermöglicht, dass in den Inkontinenzartikel aufgenommene Körperflüssigkeit über einen relativ weit in Längsrichtung erstreckten Bereich hinweg in ihrem pH-Wert reguliert werden kann, beispielsweise im Fall eines relativ großen Auftreffbereichs oder größerflächiger Erstausbreitung einer größeren Flüssigkeitsmenge an der hautseitigen Oberfläche des Inkontinenzartikels.

Bevorzugt sind der erste Bereich und/oder der zweite Bereich zumindest teilweise in dem Schrittabschnitt des Inkontinenzartikels verortet. Beispielsweise kann bei einer Anordnung des ersten Bereichs und/oder zweiten Bereichs in dem Schrittabschnitt des Inkontinenzartikels, also in einem zwischen den Beinen des Nutzers zu liegen kommenden und/oder einen Miktionsbereich des Inkontinenzartikels umfassenden oder sich über den Miktionsbereich hinweg erstreckenden Abschnitt des Inkontinenzartikels, die Körperflüssigkeit über eine Längserstreckung des Abschnitts oder Miktionsbereichs hinweg rasch mit dem pH-Regulationsmittel in Kontakt treten. Der Begriff "Miktionsbereich" bezeichnet im Rahmen dieser Erfindung einen Bereich des wahrscheinlichen Auftreffens von Körperflüssigkeit in der Gebrauchssituation, insbesondere von Urin bei der Miktion.

In einem bevorzugten Ausführungsbeispiel weist die jeweilige Längserstreckung des ersten Bereichs und/oder des zweiten Bereichs einen Wert von 10-100 %, insbesondere 15-80 %, weiter insbesondere 25-75 % einer Längserstreckung des Absorptionskörpers auf. Die Längserstreckung des Absorptionskörpers ist eine maximale Erstreckung des Absorptionskörpers von einem vordersten Ende zu einem hintersten Ende des Absorptionskörpers gemessen in der Längsrichtung des Inkontinenzartikels. Eine derartig dimensionierte Längserstreckung des ersten Bereichs und/oder zweiten Bereichs verbessert auf vorteilhafte Weise eine gleichmäßigere pH-Regulation in der Längsrichtung des Inkontinenzartikels, insbesondere auch in weiter von dem Miktionsbereich entfernten Bereichen.

Die Erfinder haben festgestellt, dass Inkontinenzartikel vorsorglich zur Vorbeugung eines möglichen Auslaufens häufig vor Erreichen einer hohen Füllmenge gewechselt werden, jedoch aus Kostenspargründen ein zu häufiger Wechsel von den Nutzern vermieden wird. Daher kann in verschiedenen Anwendungssituationen eine in einem mittleren Wertebereich angesiedelte Längserstreckung des ersten Bereichs und oder zweiten Bereichs relativ zur Längserstreckung des Absorptionskörpers vorteilhaft sein.

Im Fall einer länger dauernden Nutzung des jeweiligen Inkontinenzartikels bis zum Erreichen einer hohen Füllmenge, beispielsweise in der Nacht, kann eine Längserstreckung des ersten und/oder zweiten Bereichs über einen eher großen Teil der Längserstreckung des Absorptionskörpers, mithin eine Anordnung von pH-Regulationsmittel über einen relativ großen Bereich hinweg, die Gefahr durch Hautreizungen verringern. Im Fall einer Anordnung des pH-Regulationsmittels über 100% der Längserstreckung des Absorptionskörpers, also einer kontinuierlichen Anordnung in Längsrichtung über den gesamten Absorptionskörper hinweg, ergeben sich außerdem prozesstechnische Vorteile gegenüber einer diskontinuierlichen Anordnung.

Eine eher geringe Längserstreckung des ersten Bereichs und/oder zweiten Bereichs relativ zur Längserstreckung des Absorptionskörpers kann besonders in Situationen mit häufigem Wechsel des Inkontinenzartikels, beispielsweise während des Tages oder bei häufigen Miktionen mit jeweils geringem Flüssigkeitsvolumen, pH-Regulationsmittel in einem kleineren Abschnitt des Inkontinenzartikels vorgesehen sein, wobei auf vorteilhafte Weise die Kosten pro Inkontinenzartikel gesenkt werden können und gleichzeitig die Gefahr von Hautreizungen nicht oder nicht über Gebühr erhöht ist. So kann der Nutzer über eine längere Zeit betrachtet von einer vorteilhaften pH-Regulation profitieren bei relativ geringen Gesamtkosten.

In einer alternativen Ausführungsform kann bei einer Längserstreckung des ersten Bereichs und des zweiten Bereichs von weniger als 100 %, insbesondere weniger als 90 %, weiter insbesondere weniger als 80 %, weiter insbesondere weniger als 70 % der Längserstreckung des Absorptionskörpers, ein hinterer, in Längsrichtung zwischen dem ersten Bereich und/oder dem zweiten Bereich und einem hinteren Ende des Inkontinenzartikels angeordneter Abschnitt des Absorptionskörpers, und/oder ein vorderer, in Längsrichtung zwischen dem ersten Bereich und/oder dem zweiten Bereich und einem vorderen Ende des Inkontinenzartikels angeordneter Abschnitt des Absorptionskörpers pH-Regulationsmittel aufweisen.

Dadurch können auch Teilmengen von Urin rascher pH-reguliert werden, die sich bei einem schwallartigen Auftreffen gegebenenfalls zunächst an der hautseitigen Oberfläche des Inkontinenzartikels ausbreiten und erst im hinteren oder vorderen Abschnitt des Absorptionskörpers aufgenommen werden.

Es ist jedoch auch denkbar, dass der vordere und/oder hintere Abschnitt des Absorptionskörpers im Wesentlichen frei von pH-Regulationsmittel ist.

In einer bevorzugten Ausführungsform ist eine Längserstreckung des zentralen Bereichs mindestens gleich groß wie die Längserstreckung des ersten Bereichs und/oder des zweiten Bereichs, und/oder gleich groß wie die Längserstreckung des Absorptionskörpers. Auf vorteilhafte Weise wird damit eine Anordnung von pH-Regulationsmittel in einem in Querrichtung mittig des Inkontinenzartikels zu verortenden Bereich, mithin einem Bereich der leicht mit häufig empfindlicheren Schleimhäuten des Nutzers in näheren Kontakt treten kann, zumindest verringert, wodurch die Gefahr von Irritationen dieser Hautareale reduziert sein kann.

Vorzugsweise ist das pH-Regulationsmittel zwischen dem Topsheet und dem Absorptionskörper angeordnet. Hierbei wird auf vorteilhafte Weise ein rasches In-Kontakt-Treten der in den Inkontinenzartikel aufgenommenen Körperflüssigkeiten mit dem pH-Regulationsmittel erleichtert, so dass eine Kontrolle des pH-Werts bereits vor Erreichen eines dauerhaften Speicherortes in Gang gesetzt werden kann. Das Topsheet kann dabei auf vorteilhafte Weise ein vorzeitiges Austreten des pH-Regulationsmittels und damit einen Funktionsverlust vor dem eigentlichen Gebrauch des Inkontinenzartikels vermeiden helfen. Weiter kann das Topsheet als Trennschicht zwischen der Haut des Nutzers und dem pH-Regulationsmittel dabei unterstützen, dass nicht bereits vor einer Miktion, beispielsweise durch geringe Mengen an Hautschweiß, das pH-Regulationsmittel vorzeitig aktiviert wird und unter Umständen der pH-Wert der Haut auf ungünstige Weise beeinflusst werden kann.

Bevorzugt ist das pH-Regulationsmittel herstellerseitig auf einer im Gebrauch körperzugewandten Oberseite des Absorptionskörpers oder einer im Gebrauch körperzugewandten Oberseite der ersten Saugkörperlage angeordnet. Das pH-Regulationsmittel kann alternativ herstellerseitig auf eine im Gebrauch körperabgewandte Oberseite des Topsheets appliziert sein. Denkbar ist auch, dass das pH-Regulationsmittel herstellerseitig im Inneren des Absorptionskörpers oder der ersten Saugkörperlage, dabei entlang einer Dickenrichtung des Inkontinenzartikels gleichförmig oder ungleichförmig verteilt angeordnet wird. Die Dickenrichtung des Inkontinenzartikels verläuft senkrecht zur Längsrichtung und zur Querrichtung des Inkontinenzartikels.

In einem bevorzugten Ausführungsbeispiel weist der Absorptionskörper zumindest eine erste Saugkörperlage auf. Weiter bevorzugt weist der Absorptionskörper eine erste Saugkörperlage und eine zwischen der ersten Saugkörperlage und dem Backsheet angeordnete zweite Saugkörperlage auf. Hierdurch kann im Gebrauch aufgenommene und mit pH-Regulationsmittel in Kontakt getretene oder dann in Kontakt tretende Körperflüssigkeit auf vorteilhafte Weise relativ rasch ins Innere des Absorptionskörpers geleitet und dort gesammelt werden, wobei sich ein zunehmend gleichmäßigerer erwünschter pH-Wert einstellen kann.

Der Absorptionskörper des Inkontinenzartikels ist geeignet und dazu bestimmt Körperflüssigkeiten, insbesondere Urin aufzunehmen und dauerhaft zu speichern. Der Begriff "Saugkörperlage" bezeichnet dabei eine Lage des Absorptionskörpers, der die eigentliche Aufgabe der Absorption, mithin der zumindest kurzfristigen insbesondere der dauerhaften Speicherung von Körperflüssigkeiten zukommt. Dazu kann der Absorptionskörper und/oder die erste Saugkörperlage und/oder die zweite Saugkörperlage ein oder mehrere absorbierende Materialien wie Zellstofffasern ("fluff") oder Kunststofffasern aufweisen oder daraus gebildet sein.

In einer bevorzugten Ausführungsform weist der Absorptionskörper, insbesondere die erste Saugkörperlage superabsorbierendes Polymer auf. Superabsorbierendes Polymer bietet den Vorteil, relativ zu anderen absorbierenden Materialien wie beispielsweise Zellulosefasern große Mengen an Flüssigkeit speichern zu können. Zudem kann superabsorbierendes Polymer die gespeicherte Flüssigkeit dauerhaft, auch unter bis zu einem gewissen Grad erhöhtem Druck, binden. Daher hilft das superabsorbierende Polymer im Absorptionskörper und/oder in der ersten Saugkörperlage dabei, einen länger andauernden Kontakt von Körperflüssigkeiten und der Haut des Nutzers und damit die Gefahr von Hautirritationen zu verringern.

Bevorzugt enthält der Absorptionskörper superabsorbierendes Polymer zu 5 - 100 Gewichtsprozent, vorzugsweise zu 10 - 95 Gewichtsprozent, weiter vorzugsweise zu 15 - 90 Gewichtsprozent, weiter vorzugsweise zu 20 - 80 Gewichtsprozent. Vorzugsweise kann das superabsorbierende Polymer zumindest das 15-fache seines Gewichts, insbesondere zumindest das 20-fache seines Gewichts, weiter bevorzugt zumindest das 30-fache seines Gewichts an 0,9 gewichtsprozentiger Kochsalzlösung absorbieren (gemessen nach NWSP 242.0. R2(15)).

Das superabsorbierende Polymer kann beispielsweise partikelförmig oder faserförmig oder blatt- oder schaumförmig ausgebildet sein. Das superabsorbierende Polymer kann weiterhin ein im Wesentlichen sortenreines Polymermaterial oder eine Mischung von zwei oder mehreren Polymermaterialien umfassen oder daraus bestehen.

Die zweite Saugkörperlage bewirkt auf vorteilhafte Weise einen höheren Tragekomfort, insbesondere bei einem am Körper näher anliegenden Inkontinenzartikel, da von einer Backsheetseite her ein weicherer haptischer Eindruck des Inkontinenzartikels und damit eine verringerte Gefahr von zum Beispiel durch Hartstellen des Inkontinenzartikels bedingten Hautreizungen befördert wird.

Bevorzugt weist die zweite Saugkörperlage weniger als 20 Gewichtsprozent, weiter bevorzugt weniger als 15 Gewichtsprozent, weiter bevorzugt weniger als 10 Gewichtsprozent, weiter bevorzugt weniger als 5 Gewichtsprozent superabsorbierendes Polymer auf, insbesondere ist die zweite Saugkörperlage frei von superabsorbierendem Polymer. Dem Fachmann ist hinreichend bekannt, dass partikelförmiges Material wie superabsorbierendes Polymer abrasive Kräfte ausüben kann, die das im Wesentlichen flüssigkeitsundurchlässige Backsheet, insbesondere eine flüssigkeitsundurchlässige Backsheetfolie des Inkontinenzartikels unter bestimmten Umständen beschädigen und zu Undichtigkeit führen können. Daher bietet die zweite Saugkörperlage auch den Vorteil, dass die Gefahr des unerwünschten Austritts von Körperflüssigkeit und dadurch gegebenenfalls drohende Hautreizungen weiter verringert sein können.

Der Absorptionskörper kann rechteckig, dreieckig, oval, T-förmig, sanduhrförmig, Y-förmig, anatomisch, asymmetrisch oder in einer anderen dem Fachmann als geeignet erscheinenden Form ausgebildet sein.

Hierbei ist zu erwähnen, dass der Absorptionskörper und/oder die erste Saugkörperlage und/oder die zweite Saugkörperlage eine gleichförmige Dicke und/oder eine gleichförmige Dichte aufweisen kann. Ebenso ist denkbar, dass der Absorptionskörper und/oder die erste Saugkörperlage und/oder die zweite Saugkörperlage ein Dicken- oder Höhenprofil oder ein Dichteprofil aufweist.

Denkbar ist weiterhin, die erste und/oder die zweite Saugkörperlage des Absorptionskörpers durch Anordnung von jeweils ein oder mehreren Schichten verschiedenen absorbierenden Materials, insbesondere aus Fasermaterial auszubilden. Im Fachgebiet hinreichend bekannt sind außerdem Kernumhüllungen aus Flachmaterialien, beispielsweise Fasermaterialien künstlichen und/oder natürlichen Ursprungs.

In einem vorteilhaften Ausführungsbeispiel umfasst der Inkontinenzartikel eine Flüssigkeitsaufnahme- und/oder -verteilungsschicht, wobei die Flüssigkeitsaufnahme- und/oder -verteilungsschicht insbesondere zwischen dem Topsheet und dem Absorptionskörper angeordnet ist. Die Flüssigkeitsaufnahme- und/oder -verteilungsschicht unterstützt eine relativ rasche Wegleitung von Körperflüssigkeiten von der hautseitigen Oberfläche des Inkontinenzartikels hin zum Absorptionskörper und/oder der ersten Saugkörperlage. Zudem kann die zwischen dem Topsheet und dem Absorptionskörper angeordnete Flüssigkeitsaufnahme- und/oder -verteilungsschicht als ein Abstandshalter zwischen der im Absorptionskörper oder in der ersten Saugkörperlage gesammelten Körperflüssigkeit und der hautseitigen Oberfläche des Inkontinenzartikels dienen, so dass auf vorteilhafte Weise die Gefahr von Hautirritationen weiter verringert sein kann.

Flüssigkeitsaufnahme- und/oder -verteilungsschichten sind im Fachgebiet bereits grundsätzlich bekannt und bestehen typischerweise aus einem Fasermaterial, insbesondere einem Vliesmaterial.

Die Fasern können natürlichen oder künstlichen Ursprungs sein und können von definierter Länge (Stapelfasern) oder kontinuierlich ("endlos") sein oder in-situ gebildet werden. Die Fasern können aus einem einzelnen Polymer oder einer Polymermischung (Einkomponentenfaser) oder aus mehr als einem einzelnen Polymer und/oder einer Polymermischung (Mehrkomponentenfaser) ausgebildet sein.

Eine Mehrkomponentenfaser weist einen Querschnitt auf, der mehr als einen einzelnen Abschnitt umfasst, wobei jeder dieser Abschnitte ein anderes Polymer oder eine andere Polymermischung umfasst oder daraus besteht. Der Begriff Mehrkomponentenfaser umfasst, ist aber nicht beschränkt auf, eine Zweikomponentenfaser. Die verschiedenen Komponenten von Mehrkomponentenfasern sind in im Wesentlichen unterschiedlichen Bereichen über den Querschnitt der Faser angeordnet und erstrecken sich häufig kontinuierlich über die Länge der Faser. Eine Mehrkomponentenfaser kann einen Gesamtquerschnitt aufweisen, der Teilbereiche der zwei oder mehr unterschiedlichen Komponenten jeglicher Form oder Anordnung aufweist, einschließlich beispielsweise koaxialer Unterabschnitte, Kern- und Hüllenunterabschnitte, nebeneinander liegende Unterabschnitte, radiale Unterabschnitte, inselförmige Unterabschnitte, etc.

Eine Zweikomponentenfaser mit einer "Kern/Hüll-Struktur" hat einen Querschnitt, der Folgendes umfasst: zwei diskrete Abschnitte, von denen jeder aus einem Polymer oder einer Polymermischung besteht, worin das Hüllpolymer oder die Hüllpolymermischkomponente um das Kernpolymer oder die Kernpolymermischungskomponente herum angeordnet ist.

Das Flächengewicht von Vliesmaterialien wird in der Regel in Gramm pro Quadratmeter (g/m²) angegeben.

Die Flüssigkeitsaufnahme- und/oder -verteilungsschicht kann derart ausgebildet sein, dass sie auf vorteilhafte Weise eine rasche Flüssigkeitsaufnahme von das Topsheet passierenden Körperflüssigkeiten oder eine verbesserte Flüssigkeitsverteilung in der Flächenrichtung oder beides unterstützt. Dazu kann die Flüssigkeitsaufnahme- und/oder -verteilungsschicht einlagig oder mehrlagig, beispielsweise zweilagig mit einer Flüssigkeitsaufnahmelage und einer Flüssigkeitsverteilungslage, ausgebildet sein. Bevorzugt ist die Flüssigkeitsaufnahme- und/oder -verteilungsschicht einlagig, also aus einem im Wesentlichen einheitlichen Material, ausgebildet.

In einer bevorzugten Ausführungsform ist die Flüssigkeitsaufnahme- und/oder - verteilungsschicht als ein Vliesmaterial aus künstlichen Fasern ausgebildet und weist weiter bevorzugt ein Flächengewicht von 10 - 80 g/m², insbesondere 15 - 70 g/m², weiter insbesondere 20 - 60 g/m², weiter insbesondere 30 - 50 g/m² auf. Die Erfinder haben nämlich festgestellt, dass künstliche Faservliese aufgrund ihrer häufig eher hydrophoben Eigenschaften dahingehend vorteilhaft wirken können, dass einmal aufgenommene Körperflüssigkeit besser im Innern des Inkontinenzartikels verbleiben kann. Bei dem bevorzugten Flächengewicht kann die Flüssigkeitsaufnahme- und/oder -verteilungsschicht auf vorteilhafte Weise einem übermäßigen Rückfluss von Körperflüssigkeit in Richtung des Topsheets entgegenwirken, ohne dabei über Gebühr in der Dickenrichtung aufzutragen oder eine vom Nutzer beim Tragen wahrgenommene Weichheit des Inkontinenzartikels zu beeinträchtigen. Daher wird auch hierdurch eine Gefahr von Hautirritationen durch andauernden Kontakt mit Körperflüssigkeiten wie Urin oder durch eine gewisse Rauheit des Inkontinenzartikels weiter reduziert.

In einer bevorzugten Ausführungsform umfasst die Flüssigkeitsaufnahme- und/oderverteilungsschicht Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisförmigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP.

Als eine Alternative ist es im Fachgebiet bekannt, ein Material aus Zellulosefasern, insbesondere aus chemisch modifizierten Zellulosefasern, beispielsweise quervernetzten Zellulosefasern, als Flüssigkeitsaufnahme- und/oder -verteilungsschicht zu verwenden.

Die Flüssigkeitsaufnahme- und/oder -verteilungsschicht kann auch andere Materialien wie perforierte Folien oder Schäume oder dergleichen umfassen oder daraus bestehen.

Die Flüssigkeitsaufnahme- und/oder -verteilungsschicht kann den Absorptionskörper im Wesentlichen vollständig oder nur teilweise überfangen, mithin im Wesentlichen eine gleiche flächenhafte Erstreckung oder eine zumindest bereichsweise geringere Erstreckung als die erste Saugkörperlage und/oder die zweite Saugkörperlage des Absorptionskörpers in Längs- und/oder Querrichtung des plan aufliegenden Inkontinenzartikels aufweisen. Bevorzugt überfängt die Flüssigkeitsaufnahme- und/oder -verteilungsschicht die erste Saugkörperlage und/oder die zweite Saugkörperlage des Absorptionskörpers zu 5 - 100%, insbesondere zu 10 - 90%, weiter insbesondere zu 15 - 80%, weiter insbesondere zu 20 - 70%, weiter insbesondere zu 25 - 60%, weiter insbesondere zu 30 - 50% ihrer flächenhaften Erstreckung.

Vorzugsweise ist die Flüssigkeitsaufnahme- und/oder -verteilungsschicht zumindest in dem Miktionsbereich angeordnet. Insbesondere erstreckt sich die Flüssigkeitsaufnahme- und/oder -verteilungsschicht über und im Bereich der Längsmittelachse des Absorptionskörpers. Weiter insbesondere erstreckt sich die Flüssigkeitsaufnahme- und/oder -verteilungsschicht über und im Bereich einer Quermittelachse des Absorptionskörpers.

In einem bevorzugten Ausführungsbeispiel ist mindestens 30 Gewichtsprozent, insbesondere mindestens 40 Gewichtsprozent, weiter insbesondere mindestens 50 Gewichtsprozent, weiter insbesondere mindestens 60 Gewichtsprozent, weiter insbesondere mindestens 70 Gewichtsprozent, weiter insbesondere mindestens 80 Gewichtsprozent, weiter insbesondere mindestens 90 Gewichtsprozent, weiter insbesondere 100 Gewichtsprozent des pH-Regulationsmittels zwischen der Flüssigkeitsaufnahme- und/oder - verteilungsschicht und dem Backsheet angeordnet. Daraus ergibt sich in vorteilhafter Weise, dass aufgenommene Körperflüssigkeit bereits nach Passieren der Flüssigkeitsaufnahme- und/oder -verteilungsschicht mit dem pH-Regulationsmittel in Kontakt treten und der pH-Wert recht rasch und effektiv reguliert werden kann.

In einem bevorzugten Ausführungsbeispiel ist das pH-Regulationsmittel zwischen der Flüssigkeitsaufnahme- und/oder -verteilungsschicht und der ersten Saugkörperlage angeordnet. Weiter bevorzugt ist das pH-Regulationsmittel zwischen der Flüssigkeitsaufnahme- und/oder -verteilungsschicht und dem Absorptionskörper angeordnet. Bei einer Anordnung des pH-Regulationsmittels zwischen zwei Komponenten des Inkontinenzartikels, wie beispielsweise Topsheet und Absorptionskörper, Flüssigkeitsaufnahme- und/oder -verteilungsschicht und erster Saugkörperlage oder Flüssigkeitsaufnahme- und/oder -verteilungsschicht und Absorptionskörper, bezieht sich die Anordnung des pH-Regulationsmittels auf die herstellerseitig vorgesehene Anordnung, mithin auf den Ort der herstellerseitigen Applikation des pH-Regulationsmittels im Inkontinenzartikel. Da der Absorptionskörper und/oder die erste Saugkörperlage zur Erfüllung ihrer vorgesehenen Funktion typischerweise Hohlräume aufweist ist es möglich, dass eine geringe Menge des applizierten pH-Regulationsmittels von einem vorgesehenen Applikationsort, insbesondere in der Dickenrichtung des Inkontinenzartikels, migrieren kann. Solchenfalls ist der herstellerseitige Applikationsort maßgeblich.

Eine Gesamtmenge an pH-Regulationsmittel pro Inkontinenzartikel, der insbesondere zur Benutzung durch Erwachsene vorgesehen ist, beträgt vorzugsweise 0,05 bis 3,00 g, weiter vorzugsweise 0,05 bis 2,50 g, weiter vorzugsweise 0,10 bis 2,00 g, weiter vorzugsweise 0,15 bis 1,50 g, weiter vorzugsweise 0,20 bis 1,00 g, weiter vorzugsweise 0,25 bis 0,80 g, weiter vorzugsweise 0,30 bis 0,70 g.

In einem bevorzugten Ausführungsbeispiel weist der Absorptionskörper mindestens einen in der Längsrichtung orientierten Kanal, insbesondere genau einen in der Längsrichtung orientierten Kanal auf. Ein Kanal ist als "in der Längsrichtung orientiert" zu betrachten, wenn der Kanal in der Längsrichtung des Inkontinenzartikels weiter erstreckt ist als in der Querrichtung des Inkontinenzartikels.

In einer alternativen Ausführungsform weist der Absorptionskörper 2 - 5, weiter insbesondere genau 2 Kanäle auf.

Der eine Kanal oder die Kanäle dienen jeweils der rascheren Aufnahme von größeren Mengen an Körperflüssigkeiten wie Urin und deren gerichtete Weiterleitung in vom Miktionsbereich weiter entfernte Regionen des Absorptionskörpers. Dadurch kann die Körperflüssigkeit insgesamt schneller absorbiert werden, so dass eine Kontaktdauer mit der Haut des Nutzers auf vorteilhafte Weise verkürzt sein kann und eine pH-Regulation rascher erfolgen kann.

In einem bevorzugten Ausführungsbeispiel ist der mindestens eine Kanal oder der genau eine Kanal symmetrisch, insbesondere spiegelsymmetrisch mit der Längsmittelachse des Absorptionskörpers als Symmetrieachse ausgebildet. Rein vorsorglich zur Vermeidung von eventuellen Unklarheiten sei angemerkt, dass die Formulierung "der mindestens eine Kanal" generell als "mindestens einer der Kanäle" zu verstehen ist. Mit Bezug zu diesem Ausführungsbeispiel wird hiermit näher ausgeführt, dass in dem Fall dass der Absorptionskörper mehr als einen Kanal aufweist, mindestens einer der vorhandenen Kanäle symmetrisch, insbesondere spiegelsymmetrisch mit der Längsmittelachse des Absorptionskörpers als Symmetrieachse ausgebildet ist.

Durch die symmetrische Ausbildung wird eine gleichmäßigere Flüssigkeitsverteilung auf die beidseits der Längsmittelachse verorteten Hälften des Absorptionskörpers und eine insgesamt raschere Absorption und pH-Regulation der Körperflüssigkeit verbessert. Zudem kann auch der Tragekomfort des Nutzers durch die hierbei erwünschte gleichmäßigere Flüssigkeitsverteilung gesteigert sein.

Bevorzugt ist mindestens ein Kanal oder der genau eine Kanal parallel zu der Längsmittelachse des Absorptionskörpers und/oder einer Längsmittelachse der ersten Saugkörperlage und/oder einer Längsmittelachse des Inkontinenzartikels angeordnet. Insbesondere ist mindestens ein Kanal oder der genau eine Kanal auf der jeweiligen Längsmittelachse des Inkontinenzartikels und/oder des Absorptionskörpers und/oder der ersten Saugkörperlage angeordnet.

Mindestens ein Kanal oder der genau eine Kanal ist auf bevorzugte Weise in der Querrichtung des Absorptionskörpers und/oder der ersten Saugkörperlage zentriert angeordnet.

Vorteilhafterweise ist mindestens ein Kanal oder der genau eine Kanal in Längsrichtung des Inkontinenzartikels zumindest teilweise in dem Miktionsbereich angeordnet.

Weiter bevorzugt erstreckt sich der mindestens eine Kanal oder der genau eine Kanal in vorteilhafter Weise über die Quermittelachse des Absorptionskörpers hinweg.

In einer bevorzugten Ausführungsform erstreckt sich mindestens ein Kanal oder der genau eine Kanal geradlinig in der Längsrichtung des Inkontinenzartikels und/oder des Absorptionskörpers und/oder der ersten Saugkörperlage. Dadurch kann die Flüssigkeit sich im Kanal ungehinderter in der Längsrichtung des Inkontinenzartikels ausbreiten.

Jedoch sind auch wellenförmige, gezackte oder bogenförmige oder jede andere Kanalform denkbar, die der Fachmann für sinnvoll erachtet.

Unterschiedliche Kanalformen wie beispielsweise rechteckig, mandelförmig, knochenförmig, T-förmig, Y-förmig, U-förmig, kurvenförmig, dreieckig, oval, sternförmig, strahlenförmig oder verzweigt sind im Stand der Technik hinreichend bekannt und von der vorliegenden Erfindung umfasst.

Die Erfinder haben festgestellt, dass für eine rasche Flüssigkeitsleitung in Längsrichtung jedoch gerade solche Kanäle vorteilhaft sind, deren Erstreckung in der Längsrichtung des Inkontinenzartikels größer ist als deren Erstreckung in Querrichtung. Insbesondere rechteckige, mandelförmige oder ovale Kanäle haben sich als vorteilhaft erwiesen.

In einer bevorzugten Ausführungsform ist der mindestens eine Kanal oder der genau eine Kanal in der ersten Saugkörperlage angeordnet und umfasst insbesondere eine sich durch die erste Saugkörperlage in Dickenrichtung zumindest teilweise, insbesondere vollständig hindurcherstreckende Ausnehmung oder ist daraus gebildet.

Dabei ist der Kanal bevorzugt im Wesentlichen frei von absorbierenden Materialien. Dazu kann der Kanal durch Weglassen des absorbierenden Materials oder durch Entfernen von absorbierendem Material mittels Schneiden oder Stanzen ausgebildet sein.

Ebenso denkbar ist jedoch, dass der Kanal durch Prägen oder Komprimieren der ersten Saugkörperlage oder des Absorptionskörpers ausgebildet ist.

In einer alternativen Ausführungsform kann der mindestens eine Kanal oder der genau eine Kanal in der zweiten Saugkörperlage angeordnet sein. Solchenfalls kann der in der zweiten Saugkörperlage angeordnete mindestens eine Kanal oder genau eine Kanal bevorzugt ein oder mehrere der vorhergehend mit Bezug zu mindestens einem Kanal oder genau einem Kanal in der ersten Saugkörperlage beschriebenen Merkmale aufweisen. Es ist auch denkbar, dass sowohl die erste Saugkörperlage als auch die zweite Saugkörperlage einen oder mehrere Kanäle aufweisen.

In einer weiteren bevorzugten Ausführungsform ist der mindestens eine Kanal oder der genau eine Kanal, insbesondere der mindestens eine Kanal oder der genau eine Kanal der ersten Saugkörperlage im Wesentlichen frei von superabsorbierendem Polymer.

Das bietet den Vorteil, dass die in den Kanal aufgenommene Körperflüssigkeit nicht im Kanal vorzeitig dauerhaft gebunden wird, sondern relativ ungehindert entlang des Kanals weitergeleitet und dadurch gleichmäßiger im Absorptionskörper verteilt werden kann. Insbesondere kann eine vorzeitige Bindung der aufgenommenen Körperflüssigkeit mittels superabsorbierendem Polymer im Kanal die pH-Regulation der Körperflüssigkeit behindern oder zumindest verzögern.

In einer bevorzugten Ausführungsform ist der mindestens eine Kanal, insbesondere der genau eine Kanal, zumindest teilweise, insbesondere vollständig innerhalb des zentralen Bereichs angeordnet. Dadurch kann die typischerweise in dem in Querrichtung eher mittig gelegenen Auftreffbereich des Inkontinenzartikel anfallende Körperflüssigkeit rascher in weiter entfernte Bereiche des Absorptionskörpers geleitet und ein Anstauen von noch nicht angemessen pH-regulierter Flüssigkeit in der Nähe der Haut des Nutzers vermindert werden.

Bevorzugt ist der mindestens eine Kanal nicht in einer Projektion in einer Dickenrichtung des Inkontinenzartikels des ersten Bereichs und/oder des zweiten Bereichs angeordnet, dabei weiter bevorzugt teilweise oder vollständig in der Projektion des zentralen Bereichs angeordnet. In einer alternativen Ausführungsform ist der mindestens eine Kanal zumindest teilweise in der Projektion in der Dickenrichtung des Inkontinenzartikels des ersten Bereichs und/oder des zweiten Bereichs angeordnet. Beispielsweise kann der mindestens eine Kanal zwei oder mehr in der gleichen und/oder unterschiedlicher Richtung erstreckte Kanalabschnitte aufweisen, welche gemeinsam einen durchgängigen, also nicht unterbrochenen Kanal, beispielsweise eine gemeinsame nicht unterbrochene Ausnehmung der ersten Saugkörperlage, ausbilden. Solchenfalls kann der mindestens eine Kanal ganz oder abschnittsweise in der Längsrichtung und/oder der Querrichtung und/oder einer anderen in einer von der Längsrichtung und der Querrichtung gebildeten Ebene verlaufenden Richtung orientiert sein und dabei ganz oder zumindest teilweise mit dem ersten Bereich und/oder dem zweiten Bereich überlappend und/oder sich über den ersten Bereich und/oder den zweiten Bereich hinaus erstreckend angeordnet sein. Der mindestens eine Kanal kann sich hierbei optional in nicht in der Projektion in der Dickenrichtung des ersten Bereichs und/oder des zweiten Bereichs und/oder des zentralen Bereichs verorteten Abschnitten des Absorptionskörpers hinein erstrecken.

Der erste Bereich und/oder der zweite Bereich ist vorzugsweise außerhalb der Projektion in der Dickenrichtung des mindestens einen Kanals verortet. Alternativ kann der erste Bereich und/oder der zweite Bereich jeweils teilweise oder ganz in der Projektion in der Dickenrichtung des mindestens einen Kanals verortet sein. Hierbei kann der erste Bereich und/oder der zweite Bereich jeweils mit dem mindestens einen Kanal abschnittsweise überlappend ausgebildet sein und/oder sich in einer von der Längsrichtung und der Querrichtung gebildeten Ebene jeweils über den mindestens einen Kanal hinaus erstrecken.

Vorzugsweise weist der Kanal kein pH-Regulationsmittel auf. Da im Gebrauch die Körperflüssigkeit typischerweise nicht dauerhaft im Kanal verbleibt, ihn hingegen nur vorübergehend auf dem Weg zum eigentlichen Speicherort passiert, kann das pH-Regulationsmittel innerhalb des Kanals gegebenenfalls nicht angemessen wirken. Es dient somit vorteilhaft sowohl einer Effektivität der pH-Wert-Kontrolle bei längerer Tragedauer als auch einem sparsamen Ressourcenverbrauch, wenn das pH-Regulationsmittel nicht im Kanal selbst sondern vorrangig in Bereichen, in denen die Flüssigkeit auf Dauer gespeichert bleibt, angeordnet ist.

In einer bevorzugten Ausführungsform ist die jeweilige Längserstreckung des ersten Bereichs und/oder des zweiten Bereichs zumindest gleich groß, insbesondere größer als eine Längserstreckung des mindestens einen Kanals, derart dass ein Verhältnis der jeweiligen Längserstreckung des ersten Bereichs und/oder des zweiten Bereichs zu der Längserstreckung des mindestens einen Kanals einen Wert von mindestens 1,0, insbesondere mindestens 1,1, weiter insbesondere mindestens 1,3 und/oder einen Wert von höchstens 4,0, insbesondere höchstens 3,5, weiter insbesondere höchstens 3,0, weiter insbesondere höchstens 2,7 aufweist. Hierdurch sind auch weiter vom Auftreffbereich entfernt liegende und vom Kanal weitergeleitete Flüssigkeit aufnehmende Bereiche mit pH-Regulationsmittel ausgestattet, so dass auf vorteilhafte Weise zumindest über die gesamte Längserstreckung des Kanals, insbesondere auch darüber hinaus in in Längsrichtung vor und/oder hinter dem Kanal verorteten Bereichen des Absorptionskörpers, eine effektive pH-Regulation unterstützt wird.

In einer weiteren bevorzugten Ausführungsform überfängt die Flüssigkeitsaufnahme- und/oder -verteilungsschicht den mindestens einen Kanal oder den genau einen Kanal zumindest bereichsweise, insbesondere vollständig. Dadurch können von der Flüssigkeitsaufnahme- und/oder -verteilungsschicht aufgenommene Körperflüssigkeiten wie Urin rascher ins Innere des Inkontinenzartikels vordringen und unter Beteiligung des jeweiligen Kanals rascher von dem Absorptionskörper, insbesondere von der ersten Saugkörperlage und/oder der zweiten Saugkörperlage absorbiert werden. Zudem verringert die Flüssigkeitsaufnahme- und/oder -verteilungsschicht eine Rücknässung hin zur Haut des Nutzers in einem von dem Kanal gebildeten Bereich, insbesondere wenn geringe Flüssigkeitsmengen vor ihrer dauerhaften Absorption im Absorptionskörper eine gewisse Zeit im Kanal verbleiben.

Es hat sich als vorteilhaft erwiesen, wenn das pH-Regulationsmittel partikelförmig ausgebildet ist.

Bevorzugt weist mindestens 50 Gewichtsprozent des pH-Regulationsmittels eine Partikelgröße von 10 bis 2000 µm, insbesondere von 50 bis 1200 µm, weiter insbesondere von 80 bis 800 µm auf. Grundsätzlich sind auch pH-Regulationsmittel mit geringerer oder größerer Partikelgröße erhältlich und verwendbar. Jedoch bieten die bevorzugten Partikelgrößen den Vorteil, dass sich Partikel einer solchen Größe bei Benetzung mit Körperausscheidungen wie Urin langsamer auflösen können als pH-Regulationsmittel geringerer Partikelgröße, so dass das pH-Regulationsmittel während der Benutzung nicht so schnell ausgewaschen wird und ein pH-regulierender Effekt länger erhalten bleibt.

Ein pH-Regulationsmittel der bevorzugten Partikelgröße lässt sich insbesondere in schnell laufenden Maschinen leichter dosieren und in den Inkontinenzartikel, insbesondere in die erste Saugkörperlage einarbeiten als beispielsweise sehr feinpudrige pH-Regulationsmittel, da es im Herstellungsprozess zu weniger Staubentwicklung und damit verbundenem Materialverlust kommt.

Außerdem wird die prozesssichere Anordnung einer vorgesehenen Menge an pH-Regulationsmittel in einem vorgesehenen Zielbereich, insbesondere im ersten Bereich und/oder zweiten Bereich positiv beeinflusst.

Andererseits bieten Partikel dieser Größe auch den Vorteil, dass sie während der Benutzung des Inkontinenzartikels haptisch weniger wahrnehmbar sind als beispielsweise Granulate mit höherer Partikelgröße, wodurch der Tragekomfort des Inkontinenzartikels verbessert wird.

Alternativ zu partikelförmigem pH-Regulationsmittel kann das pH-Regulationsmittel auch in gelöster Form, insbesondere als wässrige Lösung in den Inkontinenzartikel eingebracht sein, insbesondere durch Besprühen und optional nachfolgendem Trocknen mindestens einer Komponente des Inkontinenzartikels. Als weitere Alternative kann das pH-Regulationsmittel auch als Suspension oder Paste in den Inkontinenzartikel eingebracht sein.

Körperausscheidungen wie Urin weisen typischerweise einen alkalischeren pH-Wert als die Haut auf und können deshalb zu Hautirritationen oder -entzündungen führen. Daher sind im Stand der Technik pH-Regulationsmittel für Inkontinenzartikel zur pH-Kontrolle von Körperausscheidungen beschrieben wie schwache Säuren oder Basen, teils in Kombinationen mit einem jeweiligen Salz, beispielsweise Carbonsäuren wie Citronensäure, Essigsäure, Äpfelsäure, Milchsäure und/oder deren jeweilige Salze wie beispielsweise Natriumsalze. Grundsätzlich sind alle Substanzen oder Zusammensetzungen denkbar, die eine geeignete Pufferwirkung aufweisen und bei denen eine geringe Gefahr für Hautirritationen besteht. Hierbei gilt im Rahmen der vorliegenden Erfindung, dass superabsorbierende Polymere, also insbesondere Polymere, die zumindest das 15-fache ihres Gewichts an 0,9 gewichtsprozentiger Kochsalzlösung absorbieren (gemessen nach NWSP 242.0.R2(15)), nicht als pH-Regulationsmittel zu verstehen sind.

Die Erfinder haben festgestellt, dass es diesbezüglich vorteilhaft ist, wenn das pH-Regulationsmittel ein Salz der Citronensäure, insbesondere ein Natriumsalz der Citronensäure, also Trinatriumcitrat, Mononatriumcitrat oder Dinatriumcitrat aufweist. Die Begriffe Mononatriumcitrat, Dinatriumcitrat, und Trinatriumcitrat umfassen sowohl die wasserfreie Form als auch Hydrate der jeweiligen Substanz.

In einem bevorzugten Ausführungsbeispiel weist das pH-Regulationsmittel mindestens eine Komponente ausgewählt aus der Gruppe Mononatriumcitrat, Dinatriumcitrat, Trinatriumcitrat auf.

In einer weiteren bevorzugten Ausführungsform kann das pH-Regulationsmittel eine Kombination von zwei oder mehr Citraten, insbesondere entweder Mononatriumcitrat und Dinatriumcitrat, oder Mononatriumcitrat und Trinatriumcitrat, oder Dinatriumcitrat und Trinatriumcitrat, oder Mononatriumcitrat und Dinatriumcitrat und Trinatriumcitrat aufweisen. Durch die Auswahl oder Kombination der genannten Komponenten kann eine Feineinstellung der Pufferwirkung eines Inkontinenzartikels je nach angestrebter Benutzungsdauer oder Absorptionskapazität des Artikels erreicht werden, ohne dass wie im Falle der Verwendung einer reinen Säure als pH-Regulationsmittel, wie beispielsweise Citronensäure, die Gefahr der Entstehung stark sauer reagierender Bereiche besteht.

Falls das pH-Regulationsmittel mehr als ein Citrat umfasst, weist das pH-Regulationsmittel vorzugsweise eine homogene Mischung der jeweiligen Citrate auf. Das hat den Vorteil, dass lokale Unterschiede in der Pufferwirkung des pH-Regulationsmittels vermieden werden können.

In einer bevorzugten Ausführungsform besteht das pH-Regulationsmittel aus Mononatriumcitrat oder aus Dinatriumcitrat oder aus einer Kombination von Mononatriumcitrat und Dinatriumcitrat. Die Erfinder haben festgestellt, dass mit Mononatriumcitrat und/oder Dinatriumcitrat auf vorteilhafte Weise eine relativ gleichmäßige pH-Regulation über mehrere Miktionsereignisse hinweg erreicht werden kann. Ein weiterer Vorteil bei der Verwendung von Mononatriumcitrat ist, dass beispielsweise Mononatriumcitrat weniger hygroskopisch ist als beispielsweise Citronensäure und daher kann ein unerwünschter Eintrag von Feuchtigkeit in den Inkontinenzartikel vor der Benutzung verringert werden. Gleichsam ergibt sich aufgrund dieser Eigenschaft als weiterer Vorteil eine bessere Rieselfähigkeit, so dass sich Mononatriumcitrat leichter dosieren und/oder positionssicherer applizieren lässt.

Durch die Verwendung nur einer Komponente als pH-Regulationsmittel ergeben sich zudem prozesstechnische Vorteile, da keine Vermischung verschiedener Komponenten oder Aufrechterhaltung einer homogenen Durchmischung während des Herstellungsprozesses erforderlich ist. Zudem wird eine gleichmäßige Pufferwirkung über die pH-Regulationsmittel enthaltenden Bereiche des Inkontinenzartikels hinweg weiter unterstützt.

Rein vorsorglich sei angemerkt, dass die vorgehend beschriebene Zusammensetzung des pH-Regulationsmittels aus einer oder mehreren Komponenten bezogen ist auf eine herstellerseitige Bereitstellung des pH-Regulationsmittels in einem im Wesentlichen trockenen Zustand und gegebenenfalls ohne Berücksichtigung einer chemischen Dissoziation in einer Lösung vor oder während der Gebrauchssituation.

In einer bevorzugten Ausführungsform beträgt die flächenbezogene Menge an pH-Regulationsmittel in dem ersten Bereich und/oder zweiten Bereich 5 bis 100 g/m², insbesondere 10 bis 90 g/m², weiter insbesondere 15 bis 80 g/m², weiter insbesondere 20 bis 70 g/m². Bei der genannten flächenbezogenen Menge ist auf vorteilhafte Weise ausreichend pH-Regulationsmittel für eine gute Applizierbarkeit und für eine effektive pH-Wert-Kontrolle bei üblicherweise vorgesehener Nutzung des Inkontinenzartikels vorhanden, ohne dass weit übermäßig viel pH-Regulationsmittel verbraucht wird. Daher ergibt sich eine Schonung von Ressourcen und relativ geringere Kosten.

Die Mengenangaben des pH-Regulationsmittels sind so zu verstehen, dass sie sich auf ein wasserfreies pH-Regulationsmittel, beispielsweise wasserfreies Mononatriumcitrat, beziehen. Falls das pH-Regulationsmittel ein oder mehrere Hydrate oder wie nachfolgend beschrieben mehr als 5% additive Substanzen umfasst, wird die Gesamtmenge des pH-Regulationsmittels in g/m² derart angepasst, dass die Gesamtmenge an dem enthaltenen reinen und/oder wasserfreien pH-Regulationsmittel der vorstehenden bevorzugten Menge entspricht.

Bevorzugt wird das Mononatriumcitrat oder Dinatriumcitrat oder Trinatriumcitrat im Wesentlichen in reiner Form verwendet, insbesondere mit einem Reinheitsgrad von mindestens 90%, weiter insbesondere mindestens 95%, weiter insbesondere mindestens 98%, weiter insbesondere mindestens 99%, weiter insbesondere 100%. Insbesondere wird das Mononatriumcitrat oder Dinatriumcitrat oder Trinatriumcitrat mit einer Reinheit verwendet, welche den Anforderungen der German Pharmaceutical Codex (DAC) oder der Commission Regulation (EU) 231/2012 genügt. Dadurch können durch Rückstände anderer Substanzen ausgelöste Hautirritationen bei Benutzung des Inkontinenzartikels weitestgehend vermieden werden.

Es ist jedoch auch denkbar, dass das pH-Regulationsmittel eine geringe Menge einer oder mehrerer additiver Substanzen aufweist, beispielsweise Konfektionierungs- und/oder Konditionierungsmittel wie Trennmittel, Stabilisatoren, Staubbindemittel, Antibackmittel, Netzmittel, Koagulationsmittel, Antikoagulationsmittel, adhäsive oder oberflächenaktive Substanzen oder antimikrobielle Substanzen, Farb- oder Duftstoffe oder pH-Indikatoren. Solchenfalls beträgt der Anteil der einen oder mehreren anderen Substanzen an der Gesamtmenge des pH-Regulationsmittels insgesamt bevorzugt weniger als 10%, weiter bevorzugt weniger als 5%, weiter bevorzugt weniger als 2%, weiter bevorzugt weniger als 1%.

Bei dem Inkontinenzartikel kann es sich um unterschiedliche Ausführungsformen handeln wie beispielsweise Inkontinenzvorlagen, Inkontinenzeinlagen, Slipeinlagen, Inkontinenzwindeln offenen Typs, Inkontinenzwindeln geschlossenen Typs oder auch Krankenunterlagen.

Je nach Typ oder Ausführungsform kann der Inkontinenzartikel ein oder mehrere weitere im Stand der Technik hinlänglich bekannte Merkmale aufweisen wie beispielsweise Seitenteile, herstellerseitig fest verbundene oder wiederverschließbare Bauch- und Rückenpanel, Verschlusssysteme oder-tapes, Flügel, Elastifizierungen, Auslaufschutzsysteme (z.B. Cuffs), Nässeanzeiger, Kennzeichnungsmittel, hautpflegende oder geruchsregulierende Substanzen oder Zusammensetzungen, Haft- oder Verschluss- oder Befestigungsmittel (z.B. Klebstoff, Haken, Klettverschluss) sowie andere dem Fachmann bekannte oder sinnvoll erscheinende Merkmale.

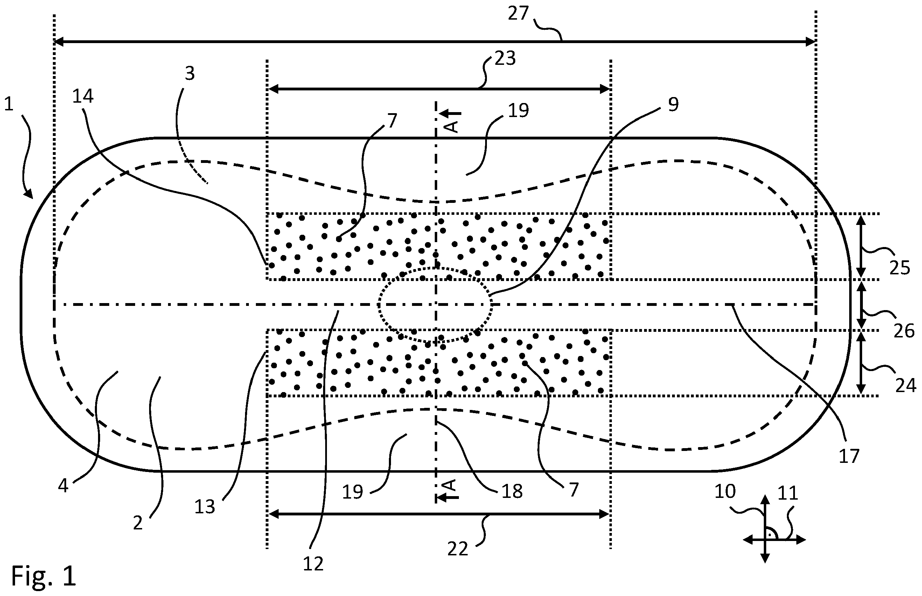

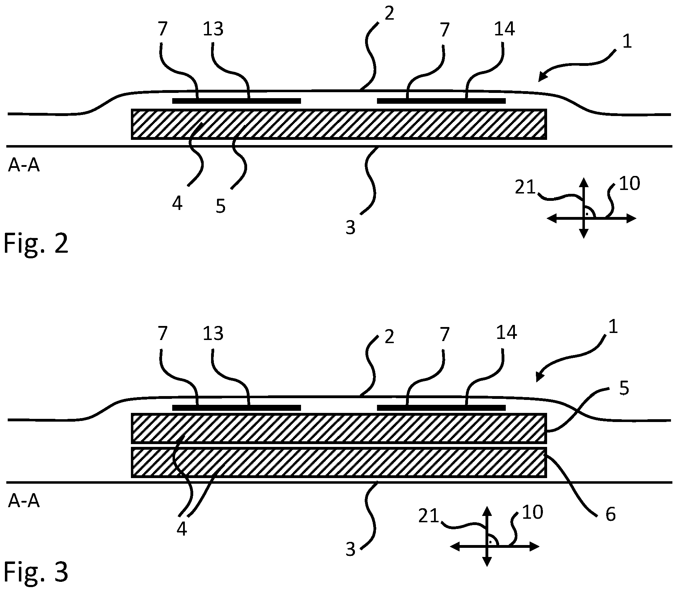

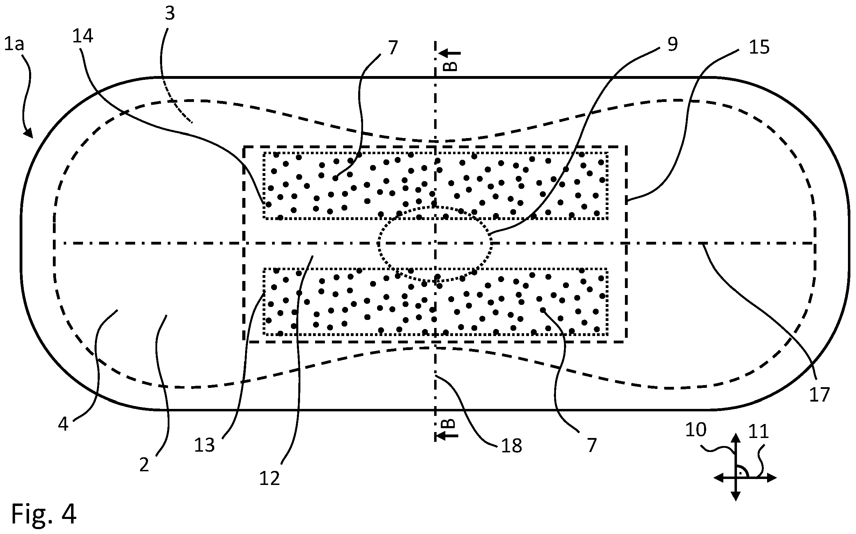

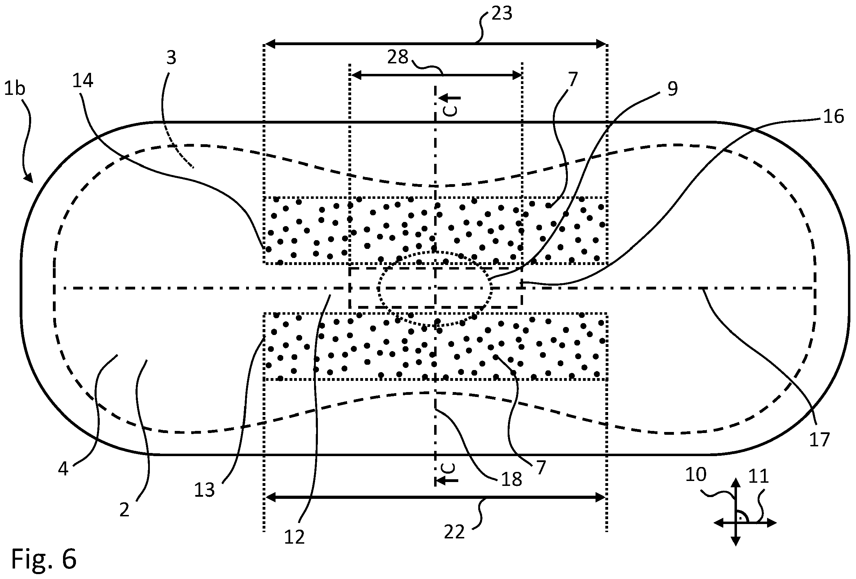

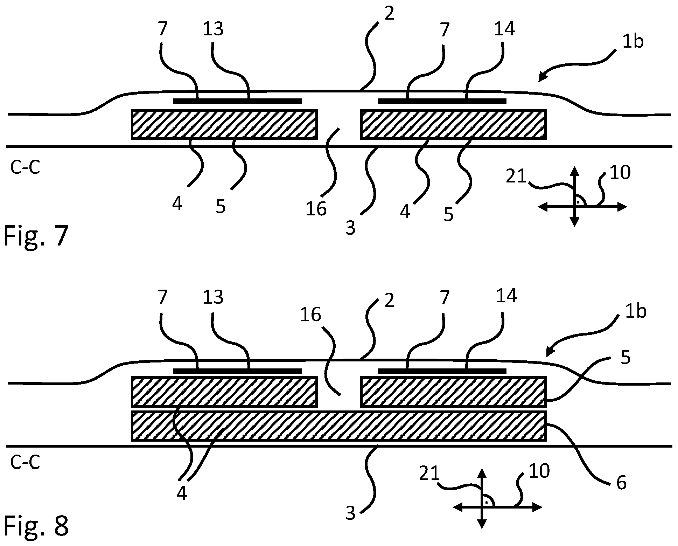

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung und Beispielen. In der Zeichnung zeigt:
**Figur 1**
   schematisch, nicht maßstäblich eine Darstellung in Draufsicht eines Inkontinenzartikels,
**Figur 2**
   schematisch, nicht maßstäblich eine Schnittansicht des Inkontinenzartikels der Figur 1 entlang einer Quermittelachse des Absorptionskörpers,
**Figur 3**
   schematisch, nicht maßstäblich eine Schnittansicht des Inkontinenzartikels der Figur 1 entlang einer Quermittelachse des Absorptionskörpers analog zu Figur 2 jedoch mit einer optionalen zweiten Saugkörperlage,
**Figur 4**
   schematisch, nicht maßstäblich eine Darstellung in Draufsicht eines Inkontinenzartikels mit einer optionalen Flüssigkeitsaufnahme- und/oder -verteilungsschicht,
**Figur 5**
   schematisch, nicht maßstäblich eine Schnittansicht des Inkontinenzartikels der Figur 4 entlang der Quermittelachse des Absorptionskörpers,
**Figur 6**
   schematisch, nicht maßstäblich eine Darstellung in Draufsicht eines Inkontinenzartikels mit einem optionalen Kanal
**Figur 7**
   schematisch, nicht maßstäblich eine Schnittansicht des Inkontinenzartikels der Figur 6 entlang der Quermittelachse des Absorptionskörpers, und
**Figur 8**
   schematisch, nicht maßstäblich eine Schnittansicht des beispielhaften Inkontinenzartikels der Figur 6 entlang der Quermittelachse des Absorptionskörpers analog zu Figur 7 jedoch mit einer optionalen zweiten Saugkörperlage.
**Figuren 9a****-c**
   jeweils schematisch, nicht maßstäblich eine Darstellung in Draufsicht eines Inkontinenzartikels mit einem jeweiligen bevorzugt ausgebildeten Kanal und außerhalb einer Projektion in Dickenrichtung des Kanals verorteten ersten Bereich und zweiten Bereich
**Figuren 10a****-f**
   jeweils schematisch, nicht maßstäblich eine Darstellung in Draufsicht eines Inkontinenzartikels mit mindestens einem jeweiligen bevorzugt ausgebildeten Kanal und teilweise in der Projektion in Dickenrichtung des Kanals angeordneten ersten Bereich und zweiten Bereich

**Figur 1** zeigt beispielhaft einen erfindungsgemäßen Inkontinenzartikel 1 für die Aufnahme von Körperausscheidungen, umfassend eine Längsrichtung 11, eine Querrichtung 10, ein zumindest bereichsweise flüssigkeitsdurchlässiges Topsheet 2, ein im Wesentlichen flüssigkeitsundurchlässiges Backsheet 3 und einen zwischen dem Topsheet 2 und dem Backsheet 3 angeordneten und eine Längsmittelachse 17 aufweisenden Absorptionskörper 4. Der Inkontinenzartikel 1 weist ein zwischen dem Topsheet 2 und dem Backsheet 3 angeordnetes pH-Regulationsmittel 7 auf. Das pH-Regulationsmittel 7 ist in einem ersten Bereich 13 und einem zweiten Bereich 14 angeordnet. Der erste Bereich 13 und der zweite Bereich 14 weist jeweils eine Längserstreckung 22,23 und eine Quererstreckung 24,25 auf. Der erste Bereich 13 und der zweite Bereich 14 sind in der Querrichtung 10 des Inkontinenzartikels 1 voneinander beabstandet derart, dass ein zwischen dem ersten Bereich 13 und dem zweiten Bereich 14 verorteter und die Längsmittelachse 17 des Absorptionskörpers 4 zumindest teilweise überfangender zentraler Bereich 12 im Wesentlichen frei von pH-Regulationsmittel ist.

Der zentrale Bereich 12 grenzt in dem in **Figur 1** dargestellten Beispiel direkt an den ersten Bereich 13 und den zweiten Bereich 14 an. Der erste Bereich 13 und der zweite Bereich 14 sind spiegelsymmetrisch beidseits der Längsmittelachse 17 des Absorptionskörpers 4 ausgebildet. Der erste Bereich 13 und der zweite Bereich 14 weisen also eine im Wesentlichen gleich große und gleichförmige Fläche auf, über die hinweg jeweils das pH-Regulationsmittel 7, dabei also beidseits der Längsmittelachse 17, bereitgestellt ist. Der erste Bereich 13 und der zweite Bereich 14 ist in diesem Beispiel jeweils streifenförmig ausgebildet, derart dass die jeweilige Längserstreckung 22,23 des ersten Bereichs 13 und des zweiten Bereichs 14 größer ist als die Quererstreckung 24,25 des jeweiligen ersten Bereichs 13 oder zweiten Bereichs 14. Die jeweilige Längs- und Quererstreckung des ersten Bereichs und des zweiten Bereichs entspricht einer jeweiligen maximalen Erstreckung des jeweiligen Bereichs. In diesem beispielhaften Inkontinenzartikel beträgt die Längserstreckung 22,23 des ersten Bereichs 13 und des zweiten Bereichs 14 jeweils 140 mm. Die Quererstreckung 24,25 des ersten Bereichs 13 und des zweiten Bereichs 14 beträgt in diesem Beispiel jeweils 27 mm. In **Figur 1** sind der erste Bereich 13 und der zweite Bereich 14 jeweils rechteckig dargestellt. Alternativ ist auch denkbar, den ersten Bereich und den zweiten Bereich in einer wie vorhergehend näher beschriebenen anderen Form, beispielsweise oval, auszubilden. Der erste Bereich 13 ist in der Längsrichtung 11 und in der Querrichtung 10 im Wesentlichen jeweils gleich weit erstreckt wie der zweite Bereich 14. Die Formulierung "im Wesentlichen gleich weit erstreckt" ist so zu verstehen, dass beispielsweise aufgrund der bei der Herstellung des Inkontinenzartikels gegebenenfalls auftretenden geringen Abweichungen der Längs- und/oder Quererstreckung der im Wesentlichen gleich weiten Erstreckung des ersten Bereichs und des zweiten Bereichs nicht entgegenstehen sollen. Dazu kann die jeweilige Längs- und/oder Querrichtung des ersten Bereichs oder des zweiten Bereichs bis zu 15 %, insbesondere bis zu 10 %, weiter insbesondere bis zu 5 % von der jeweiligen Längs- und/oder Quererstreckung des jeweiligen anderen zweiten Bereichs oder ersten Bereichs abweichen. In einer alternativen Ausführungsform können der erste Bereich und der zweite Bereich auch als in der Längs- und/oder der Querrichtung unterschiedlich weit erstreckte Bereiche vorgesehen sein.

Der erste Bereich 13 und der zweiten Bereich 14 sind in diesem beispielhaften Inkontinenzartikel 1 in einem im Gebrauch zwischen den Beinen eines Nutzers zu liegen kommenden und einen Miktionsbereich 9 des Inkontinenzartikels 1 umfassenden Schrittabschnitt 19 des Inkontinenzartikels 1 verortet. Der Absorptionskörper 4 ist sanduhrförmig ausgebildet, mithin im Schrittabschnitt 19 schmaler als in einem vorderen oder hinteren Abschnitt (ohne Bezugszeichen) ausgebildet. Alternativ kann der Absorptionskörper auch in einer anderen dem Fachmann als geeignet erscheinenden Form wie vorhergehend näher beschrieben, beispielsweise rechteckig oder oval, ausgebildet sein. Der Absorptionskörper 4 und der Inkontinenzartikel 1 sind in diesem Beispiel im Wesentlichen symmetrisch ausgebildet. Weiter ist es denkbar, den Absorptionskörper und/oder den Inkontinenzartikel asymmetrisch, also beispielsweise hinten breiter oder schmaler als vorne auszubilden.

Der zentrale Bereich 12 weist eine Quererstreckung 26 auf, die in diesem Beispiel 20 mm beträgt. Die Quererstreckung des zentralen Bereichs entspricht einem Abstand in Querrichtung von einem der Längsmittelachse des Absorptionskörpers nächstgelegenen Endes des ersten Bereichs zu einem der Längsmittelachse des Absorptionskörpers nächstgelegenen Endes des zweiten Bereichs. Der Absorptionskörper 4 des Inkontinenzartikels 1 weist eine Längserstreckung 27 von 310 mm auf. Die jeweilige Längserstreckung 22,23 des ersten Bereichs 13 und des zweiten Bereichs 14 beträgt in diesem Beispiel 45 % der Längserstreckung 27 des Absorptionskörpers 4. Der zentrale Bereich 12, welcher wie vorhergehend näher ausgeführt im Wesentlichen frei von pH-Regulationsmittel ist, weist in diesem beispielhaften Inkontinenzartikel eine Längserstreckung (in **Figur 1** nicht dargestellt) auf, welche gleich groß ist wie die Längserstreckung 27 des Absorptionskörpers 4, mithin also größer als die jeweilige Längserstreckung 22,23 des ersten Bereichs 13 und des zweiten Bereichs 14.

In dem beispielhaften Inkontinenzartikel ist das pH-Regulationsmittel 7 partikelförmig ausgebildet und besteht aus Mononatriumcitrat. Das pH-Regulationsmittel kann jedoch auch eine oder mehrere Komponenten ausgewählt aus der Gruppe Mononatriumcitrat, Dinatriumcitrat, Trinatriumcitrat aufweisen. Als weitere Alternative kann das pH-Regulationsmittel aus Dinatriumcitrat oder wie vorhergehend näher beschrieben aus einer Kombination von beispielsweise Mononatriumcitrat und Dinatriumcitrat bestehen. Das partikelförmige pH-Regulationsmittel ist in der jeweiligen Draufsicht auf den Inkontinenzartikel der **Figur 1** und der nachfolgenden **Figuren 4** **und** **6** jeweils als eine Vielzahl an Punkten dargestellt. Aus Gründen der Vereinfachung ist das pH-Regulationsmittel in den jeweiligen nachfolgenden Schnittansichten der **Figuren 2,3****,****5****,****7 und 8** jeweils als kontinuierliche Linien dargestellt.

Wie in **Figur 2** in der Schnittansicht entlang der Quermittelachse 18 des Absorptionskörpers 4 des beispielhaften Inkontinenzartikels 1 der **Figur 1** dargestellt, ist das pH-Regulationsmittel 7 zwischen dem Topsheet 2 und dem Absorptionskörper 4 angeordnet. In diesem Beispiel weist der Absorptionskörper 4 eine erste Saugkörperlage 5 auf. Der Absorptionskörper 4, mithin in diesem Beispiel die erste Saugkörperlage 5 weist superabsorbierendes Polymer (nicht dargestellt) auf. Alternativ kann der Absorptionskörper und/oder die erste Saugkörperlage ein oder mehrere absorbierende Materialien wie Zellstofffasern ("fluff") oder Kunststofffasern oder eine Mischung daraus aufweisen oder daraus gebildet sein. In einer alternativen Ausführungsform kann der Absorptionskörper 4, wie in **Figur 3** dargestellt, auch mehr als eine Saugkörperlage, hier die erste Saugkörperlage 5 und eine zwischen der ersten Saugkörperlage 5 und dem Backsheet 3 angeordnete zweite Saugkörperlage 6 aufweisen. In dieser beispielhaften Ausführungsform umfasst die erste Saugkörperlage eine Mischung aus Zellulosefasern und superabsorbierendem Polymer, und die zweite Saugkörperlage ist aus Zellulosefasern ausgebildet. In **Figur 3** sind die erste Saugkörperlage 5 und die zweite Saugkörperlage 6 in der Querrichtung 10 und in der Längsrichtung (nicht dargestellt) gleich weit erstreckt. Es ist denkbar, dass die erste Saugkörperlage alternativ in der Querrichtung und/oder in der Längsrichtung weniger weit oder weiter erstreckt ist als die zweite Saugkörperlage.

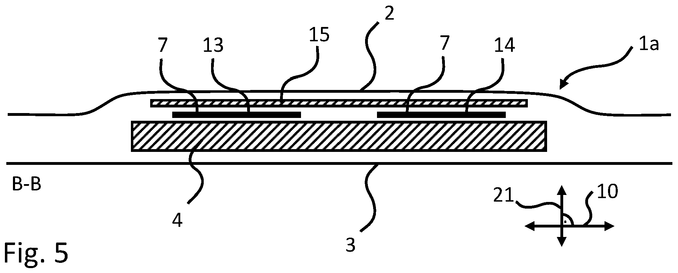

**Figur 4** zeigt einen beispielhaften Inkontinenzartikel 1a umfassend eine optionale Flüssigkeitsaufnahme- und/oder -verteilungsschicht 15. Die Flüssigkeitsaufnahme- und/oder -verteilungsschicht 15 erstreckt sich über den Miktionsbereich 9, die Längsmittelachse 17 und die Quermittelachse 18 des Absorptionskörpers 4 hinweg. Wie in **Figur 5** in der Schnittansicht des beispielhaften Inkontinenzartikels 1a der **Figur 4** dargestellt, ist die Flüssigkeitsaufnahme- und/oder -verteilungsschicht 15 in diesem Beispiel zwischen dem Topsheet 2 und dem Absorptionskörper 4 angeordnet. Die Flüssigkeitsaufnahme- und/oder - verteilungsschicht 15 besteht aus einem einlagigen kardierten und heißluftverfestigten Vliesmaterial aus künstlichen Zweikomponentenfasern (Bico/PES) mit einem Flächengewicht von 40 g/m². Wie bereits ausgeführt sind grundsätzlich auch andere vorgehend näher beschriebene Materialien zur Verwendung als Flüssigkeitsaufnahme- und/oder - verteilungsschicht denkbar. Das pH-Regulationsmittel 7 ist in diesem beispielhaften Inkontinenzartikel 1a vollständig, mithin zu 100 Gewichtsprozent zwischen der Flüssigkeitsaufnahme- und/oder -verteilungsschicht 15 und dem Backsheet 3 angeordnet. Das pH-Regulationsmittel 7 ist zwischen der Flüssigkeitsaufnahme- und/oder - verteilungsschicht 15 und dem Absorptionskörper 4 angeordnet. Eine andere Anordnung des pH-Regulationsmittels ist wie vorhergehend näher beschrieben denkbar.

**Figur 6** zeigt einen beispielhaften Inkontinenzartikel 1b umfassend einen Absorptionskörper 4 mit einem optionalen in der Längsrichtung 11 orientierten Kanal 16. Der dargestellte Inkontinenzartikel 1b weist genau einen in der Längsrichtung 11 orientierten Kanal 16 auf, der spiegelsymmetrisch mit der Längsmittelachse 17 des Absorptionskörpers 4 als Symmetrieachse ausgebildet ist. Der Kanal 16 ist vollständig innerhalb des zentralen Bereichs 12 angeordnet. Der Kanal 16 weist dabei kein pH-Regulationsmittel auf. Wie in **Figur 7** in einer Schnittansicht des beispielhaften Inkontinenzartikels 1b der **Figur 6** dargestellt, ist der Kanal 16 in der ersten Saugkörperlage 5 angeordnet. Der Kanal 16 ist gebildet aus einer sich durch die erste Saugkörperlage 5 in Dickenrichtung 21 vollständig hindurcherstreckende Ausnehmung. Alternativ ist denkbar, dass der Kanal nur teilweise in der Dickenrichtung durch die erste Saugkörperlage hindurcherstreckt ist. Es ist auch denkbar, mehr als einen Kanal in der ersten Saugkörperlage vorzusehen.

Der Kanal 16 ist in der Querrichtung 10 des Absorptionskörpers 4 und der ersten Saugkörperlage 5 zentriert angeordnet. Dabei ist - wie der **Figur 6** weiter zu entnehmen - der Kanal 16 teilweise in dem Miktionsbereich 9 angeordnet derart, dass er sich in der Längsrichtung 11 über den Miktionsbereich 9 hinweg erstreckt. In diesem Beispiel erstreckt sich der Kanal 16 über die Quermittelachse 18 des Absorptionskörpers 4 hinweg. Der Kanal 16 weist eine rechteckige und geradlinige Form auf. Alternative Formen sind wie vorhergehend näher beschrieben ebenfalls denkbar.

In einer alternativen Ausführungsform kann der den mindestens einen Kanal 16 aufweisende Absorptionskörper 4, wie in **Figur 8** dargestellt, eine zwischen der ersten Saugkörperlage 5 und dem Backsheet 3 angeordnete optionale zweite Saugkörperlage 6 aufweisen. In dieser Ausführungsform erstreckt sich der Kanal 16 durch die erste Saugkörperlage 5 in Dickenrichtung 21 vollständig hindurch. Die zweite Saugkörperlage 6 weist keinen Kanal auf. Alternativ ist denkbar, dass die zweite Saugkörperlage ebenfalls mindestens einen Kanal oder genau einen Kanal aufweist, welcher in der Dickenrichtung mit dem Kanal der ersten Saugkörperlage teilweise oder vollständig überlappen kann, oder in Querrichtung oder Längsrichtung des Inkontinenzartikels versetzt zum Kanal der ersten Saugkörperlage angeordnet ist.

Bei dem in **Figur 6** dargestellten beispielhaften Inkontinenzartikel 1b ist die jeweilige Längserstreckung 22,23 des ersten Bereichs 13 und des zweiten Bereichs 14 von jeweils 140 mm größer als eine Längserstreckung 28 des Kanals 16 von 70 mm. Ein Verhältnis der jeweiligen Längserstreckung 22,23 des ersten Bereichs 13 und des zweiten Bereichs 14 zu der Längserstreckung 28 des Kanals 16 weist einen Wert von 1,5 auf.

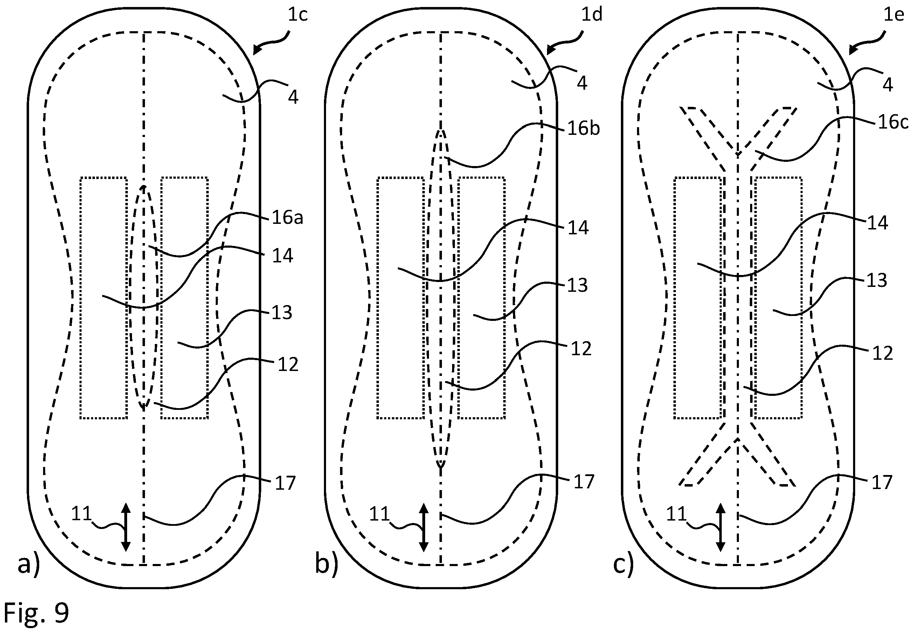

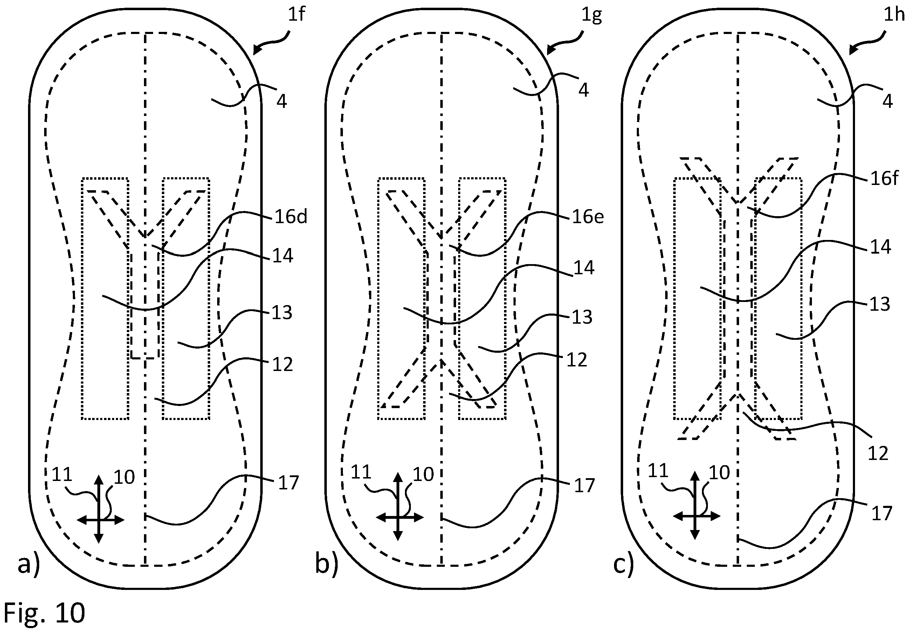

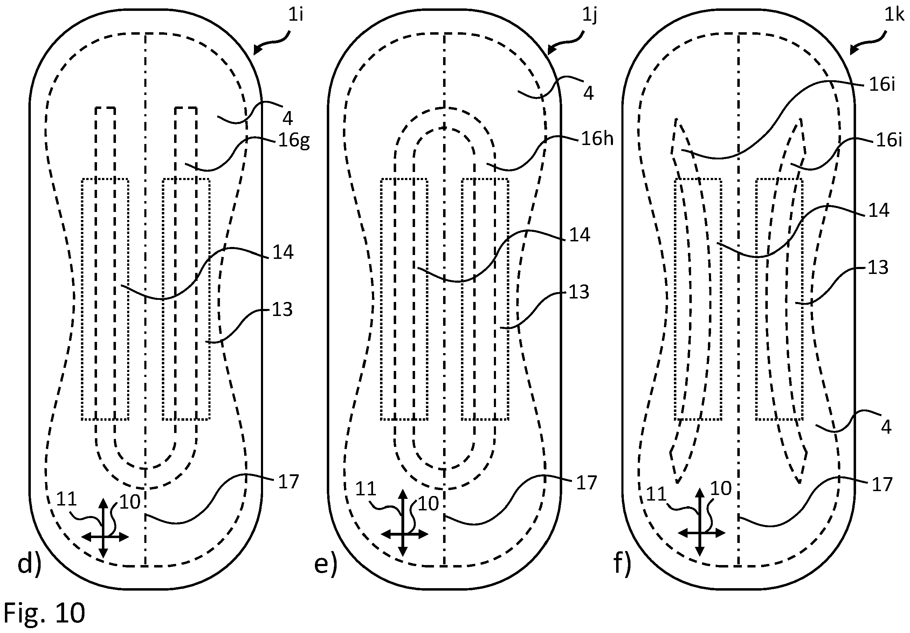

**Figuren 9** **und** **10** zeigen Inkontinenzartikel mit mindestens einem optionalen und jeweils unterschiedlich geformten Kanal. Die Darstellung der Kanalanzahl und der Kanalformen ist jeweils nicht als abschließend zu betrachten, sondern lediglich als ein Ausschnitt der von der Erfindung umfassten und vorhergehend näher beschriebenen Varianten. Aus Gründen der Übersichtlichkeit ist das pH-Regulationsmittel nicht dargestellt. Der erste Bereich 13 und der zweite Bereich 14 sind wie auch in den vorhergehenden **Figuren 1****,****4** **und** **6** jeweils mittels einer gepunkteten Umrandungslinie angedeutet. **Figuren 9a,9b,9c** zeigen jeweils schematisch, nicht maßstäblich eine Darstellung in Draufsicht eines Inkontinenzartikels 1c, 1d, 1e mit einem jeweiligen bevorzugt ausgebildeten Kanal 16a,16b,16c und außerhalb einer Projektion in Dickenrichtung (nicht dargestellt) des Kanals 16a,16b,16c verorteten ersten Bereich 13 und zweiten Bereich 14.

Der Kanal 16a in **Figur 9a** ist oval und spiegelsymmetrisch mit der Längsmittelachse 17 des Absorptionskörpers 4 ausgebildet, in der Längsrichtung 11 orientiert und vollständig innerhalb des zentralen Bereichs 12 angeordnet. Der erste Bereich 13 und der zweite Bereich 14 ist in Längsrichtung 11 weiter erstreckt als der Kanal 16a. Denkbar ist auch dass der Kanal und der erste Bereich und/oder der zweite Bereich im Wesentlichen in Längsrichtung gleich weit erstreckt sind. Der erste Bereich 13 und der zweite Bereich 14 ist jeweils außerhalb der Projektion in der Dickenrichtung (nicht dargestellt) des Kanals 16a verortet. Der Kanal 16a weist kein pH-Regulationsmittel auf.

Der Kanal 16b in **Figur 9b** ist oval und spiegelsymmetrisch mit der Längsmittelachse 17 des Absorptionskörpers 4 ausgebildet und in der Längsrichtung 11 orientiert. Der Kanal 16b ist in diesem Beispiel in Längsrichtung 11 weiter erstreckt als der erste Bereich 13 und der zweite Bereich 14. Denkbar ist auch dass der Kanal und der erste Bereich und/oder der zweite Bereich im Wesentlichen in Längsrichtung gleich weit erstreckt sind. Der erste Bereich 13 und der zweite Bereich 14 ist jeweils außerhalb der Projektion in der Dickenrichtung (nicht dargestellt) des Kanals 16b verortet. Der Kanal 16b weist kein pH-Regulationsmittel auf.

Der Kanal 16c in **Figur 9c** ist verzweigt und spiegelsymmetrisch mit der Längsmittelachse 17 des Absorptionskörpers 4 ausgebildet und in der Längsrichtung 11 orientiert. Der Kanal 16c ist in diesem Beispiel in Längsrichtung 11 weiter erstreckt als der erste Bereich 13 und der zweite Bereich 14. Denkbar ist auch dass der Kanal und der erste Bereich und/oder der zweite Bereich im Wesentlichen in Längsrichtung gleich weit erstreckt sind. Der erste Bereich 13 und der zweite Bereich 14 ist jeweils außerhalb der Projektion in der Dickenrichtung (nicht dargestellt) des Kanals 16c verortet. Der Kanal 16c weist kein pH-Regulationsmittel auf.

**Figuren 10a,10b,10c** zeigen jeweils schematisch, nicht maßstäblich eine Darstellung in Draufsicht eines Inkontinenzartikels 1f,1g,1h mit einem jeweiligen alternativen, verzweigt ausgebildeten Kanal 16d,16e,16f und teilweise in einer Projektion in Dickenrichtung (nicht dargestellt) des Kanals 16d,16e,16f verorteten ersten Bereich 13 und zweiten Bereich 14. Der Kanal 16d in **Figur 10a** und der Kanal 16e in **Figur 10b** und der Kanal 16f in **Figur 10c** ist jeweils spiegelsymmetrisch mit der Längsmittelachse 17 des Absorptionskörpers 4 ausgebildet, in der Längsrichtung 11 orientiert und teilweise innerhalb des zentralen Bereichs 12 angeordnet. Der erste Bereich 13 und der zweite Bereich 14 ist in Längsrichtung 11 weiter erstreckt als der jeweilige Kanal 16d, 16e **(****Figur 10a, Figur 10b****).** Der Kanal 16f **(****Figur 10c****)** ist in der Längsrichtung 11 weiter erstreckt als der erste Bereich 13 und der zweite Bereich 14. Der erste Bereich 13 und der zweite Bereich 14 ist jeweils teilweise in der Projektion und teilweise außerhalb der Projektion in der Dickenrichtung (nicht dargestellt) des jeweiligen Kanals 16d,16e,16f verortet (**Figur 10a, Figur 10b, Figur 10c**). Alternativ kann der erste Bereich und/oder der zweite Bereich jeweils ganz in der Projektion in der Dickenrichtung des mindestens einen Kanals verortet sein. Der erste Bereich 13 und der zweite Bereich 14 ist jeweils mit dem jeweiligen Kanal 16d,16e,16f abschnittsweise überlappend ausgebildet. Der erste Bereich 13 und der zweite Bereich 14 erstrecken sich in der von der Längsrichtung 11 und der Querrichtung 10 gebildeten Ebene jeweils über den Kanal 16d, 16e, 16f hinaus. Der Kanal 16d,16e,16f ist jeweils mit dem ersten Bereich 13 und dem zweiten Bereich 14 abschnittsweise überlappend ausgebildet. Der Kanal 16f **(****Figur 10c****)** erstreckt sich in der von der Längsrichtung 11 und der Querrichtung 10 gebildeten Ebene über den jeweiligen ersten Bereich 13 und den zweiten Bereich 14 hinaus.

**Figuren 10d,10e,10f** zeigen jeweils schematisch, nicht maßstäblich eine Darstellung in Draufsicht eines Inkontinenzartikels 1i,1j,1k mit mindestens einem jeweiligen alternativen Kanal 16g,16h,16i und teilweise in einer Projektion in Dickenrichtung (nicht dargestellt) des jeweiligen Kanals 16g,16h,16i verorteten ersten Bereich 13 und zweiten Bereich 14.

Der Kanal 16g in **Figur 10d** und der Kanal 16h in **Figur 10e** und die beiden Kanäle 16i in **Figur 10f** sind jeweils spiegelsymmetrisch mit der Längsmittelachse 17 des Absorptionskörpers 4 ausgebildet und in der Längsrichtung 11 orientiert. Der Kanal 16g **(****Figur 10d****)** ist U-förmig ausgebildet. Der Kanal 16h **(****Figur 10e****)** ist ellipsenförmig ausgebildet. Die beiden Kanäle 16i **(****Figur 10f****)** sind kurvenförmig, dabei konkav ausgebildet. Der Kanal 16g in **Figur 10d** und der Kanal 16h in **Figur 10e** und die beiden Kanäle 16i in **Figur 10f** sind in der Längsrichtung 11 weiter erstreckt als der jeweilige erste Bereich 13 und der zweite Bereich 14. Der erste Bereich 13 und der zweite Bereich 14 ist jeweils teilweise in der Projektion und teilweise außerhalb der Projektion in der Dickenrichtung (nicht dargestellt) des jeweiligen Kanals 16g,16h oder von jeweils einem der Kanäle 16i verortet **(****Figur 10d, Figur 10e, Figur 10f****).** Alternativ kann der erste Bereich und/oder der zweite Bereich jeweils ganz in der Projektion in der Dickenrichtung des mindestens einen Kanals verortet sein. Der erste Bereich 13 und der zweite Bereich 14 ist jeweils mit dem jeweiligen Kanal 16g,16h oder mit jeweils einem der Kanäle 16i abschnittsweise überlappend ausgebildet. Der erste Bereich 13 und der zweite Bereich 14 erstrecken sich in der von der Längsrichtung 11 und der Querrichtung 10 gebildeten Ebene jeweils über den Kanal 16g, 16h oder je einen der Kanäle 16i hinaus. Der Kanal 16g,16h und die Kanäle 16i sind jeweils mit dem ersten Bereich 13 und dem zweiten Bereich 14 abschnittsweise überlappend ausgebildet. Der Kanal 16g,16h und die Kanäle 16i erstrecken sich in der von der Längsrichtung 11 und der Querrichtung 10 gebildeten Ebene über den jeweiligen ersten Bereich 13 und den zweiten Bereich 14 hinaus.

In den **Figuren 9a,9b,9c****,****10a,10b,10c****,****10d,10e,10f** ist jeweils ein Kanal dargestellt. Denkbar ist auch, dass der Inkontinenzartikel ein oder mehrere weitere Kanäle aufweist. Weiter kann der Inkontinenzartikel ein oder mehrere der vorhergehend beschriebenen optionalen Merkmale wie beispielsweise eine erste und/oder zweite Saugkörperlage und/oder eine Flüssigkeitsaufnahme- und/oder -verteilungsschicht aufweisen.

Aus Gründen der Übersichtlichkeit ist in den **Figuren** jeweils ein als Inkontinenzeinlage ausgebildeter Inkontinenzartikel dargestellt. Die hierbei beschriebenen Merkmale der Erfindung sowie die optionalen Merkmale sind jedoch wie für den Fachmann leicht und eindeutig erkennbar ebenso in anderen körpernahen Inkontinenzartikeln, beispielsweise Inkontinenzvorlagen, Slipeinlagen oder Binden ohne oder mit zusätzlichen Befestigungsmitteln zur Befestigung an einem Kleidungsstück, Inkontinenzwindeln offenen Typs, Inkontinenzwindeln geschlossenen Typs, Krankenunterlagen, auf einfache Weise zu verwirklichen. Daher umfasst die Erfindung auch die genannten Arten von Inkontinenzartikeln.

Je nach Typ oder Ausführungsform kann der Inkontinenzartikel ein oder mehrere weitere im Stand der Technik hinlänglich bekannte Merkmale aufweisen wie beispielsweise Seitenteile, herstellerseitig fest verbundene oder wiederverschließbare Bauch- und Rückenpanel, Verschlusssysteme oder-tapes, Flügel, Elastifizierungen, Auslaufschutzsysteme (z.B. Cuffs), Nässeanzeiger, Kennzeichnungsmittel, hautpflegende oder geruchsregulierende Substanzen oder Zusammensetzungen, Haft- oder Verschluss- oder Befestigungsmittel (z.B. Klebstoff, Haken, Klettverschluss) sowie andere dem Fachmann bekannte oder sinnvoll erscheinende Merkmale.

## Patentansprüche

1. Inkontinenzartikel (1) für die Aufnahme von Körperausscheidungen, umfassend eine Längsrichtung (11), eine Querrichtung (10), ein zumindest bereichsweise flüssigkeitsdurchlässiges Topsheet (2), ein im Wesentlichen flüssigkeitsundurchlässiges Backsheet (3) und einen zwischen dem Topsheet (2) und dem Backsheet (3) angeordneten und eine Längsmittelachse (17) aufweisenden Absorptionskörper (4), wobei der Inkontinenzartikel (1) ein zwischen dem Topsheet (2) und dem Backsheet (3) angeordnetes pH-Regulationsmittel (7) aufweist, wobei das pH-Regulationsmittel (7) in mindestens einem ersten Bereich (13) und einem zweiten Bereich (14) angeordnet ist, wobei der erste Bereich (13) und der zweite Bereich (14) jeweils eine Längserstreckung (22,23) und eine Quererstreckung (24,25) aufweisen, wobei der erste Bereich (13) und der zweite Bereich (14) in der Querrichtung (10) des Inkontinenzartikels (1) voneinander beabstandet sind derart, dass ein zwischen dem ersten Bereich (13) und dem zweiten Bereich (14) verorteter und die Längsmittelachse (17) des Absorptionskörpers (4) zumindest teilweise überfangender zentraler Bereich (12) im Wesentlichen frei von pH-Regulationsmittel ist.

2. Inkontinenzartikel nach Anspruch 1 **dadurch gekennzeichnet, dass** der erste Bereich (13) und der zweite Bereich (14) jeweils streifenförmig ausgebildet ist, derart dass die jeweilige Längserstreckung (22,23) des ersten Bereichs (13) und des zweiten Bereichs (14) größer ist als die Quererstreckung (24,25) des jeweiligen ersten Bereichs (13) oder zweiten Bereichs (14).

3. Inkontinenzartikel nach mindestens einem der vorgenannten Ansprüche **dadurch gekennzeichnet, dass** der erste Bereich (13) und der zweite Bereich (14) spiegelsymmetrisch beidseits der Längsmittelachse (17) des Absorptionskörpers (4) ausgebildet sind.

4. Inkontinenzartikel nach mindestens einem der vorgenannten Ansprüche **dadurch gekennzeichnet, dass** die jeweilige Quererstreckung (24,25) des ersten Bereichs (13) und/oder des zweiten Bereichs (14) einen Wert von jeweils 10-60 mm, insbesondere 15-50 mm, weiter insbesondere 20-40 mm aufweist.

5. Inkontinenzartikel nach mindestens einem der vorgenannten Ansprüche **dadurch gekennzeichnet, dass** der zentrale Bereich (12) direkt an den ersten Bereich (13) und den zweiten Bereich (14) angrenzt und insbesondere eine Quererstreckung (26) des zentralen Bereichs (12) einen Wert von 3-50 mm, insbesondere 5-40 mm, weiter insbesondere 8-35 mm, weiter insbesondere 10-30 mm, weiter insbesondere 15-25 mm aufweist.

6. Inkontinenzartikel nach mindestens einem der vorgenannten Ansprüche **dadurch gekennzeichnet, dass** die jeweilige Längserstreckung (22,23) des ersten Bereichs (13) und/oder des zweiten Bereichs (14) einen Wert von mindestens 50 mm, insbesondere mindestens 60 mm, weiter insbesondere mindestens 70 mm, weiter insbesondere mindestens 80 mm, weiter insbesondere mindestens 90 mm, weiter insbesondere mindestens 100 mm aufweist.

7. Inkontinenzartikel nach mindestens einem der vorgenannten Ansprüche **dadurch gekennzeichnet, dass** die jeweilige Längserstreckung (22,23) des ersten Bereichs (13) und/oder des zweiten Bereichs (14) einen Wert von 10-100 %, insbesondere 15-80 %, weiter insbesondere 25-75 % einer Längserstreckung (27) des Absorptionskörpers (4) aufweist.

8. Inkontinenzartikel nach mindestens einem der vorgenannten Ansprüche **dadurch gekennzeichnet, dass** eine Längserstreckung des zentralen Bereichs (12) mindestens gleich groß ist wie die Längserstreckung (22,23) des ersten Bereichs (13) und/oder des zweiten Bereichs (14), und/oder gleich groß ist wie die Längserstreckung (27) des Absorptionskörpers (4).

9. Inkontinenzartikel nach mindestens einem der vorgenannten Ansprüche **dadurch gekennzeichnet, dass** das pH-Regulationsmittel (7) zwischen dem Topsheet (2) und dem Absorptionskörper (4) angeordnet ist.

10. Inkontinenzartikel nach mindestens einem der vorgenannten Ansprüche **dadurch gekennzeichnet, dass** der Absorptionskörper (4) zumindest eine erste Saugkörperlage (5), insbesondere eine erste Saugkörperlage (5) und eine zwischen der ersten Saugkörperlage (5) und dem Backsheet (3) angeordnete zweite Saugkörperlage (6) aufweist und wobei insbesondere der Absorptionskörper (4), weiter insbesondere die erste Saugkörperlage (5) superabsorbierendes Polymer aufweist.

11. Inkontinenzartikel nach mindestens einem der vorgenannten Ansprüche **dadurch gekennzeichnet, dass** der Inkontinenzartikel eine Flüssigkeitsaufnahme- und/oderverteilungsschicht (15) umfasst, wobei die Flüssigkeitsaufnahme- und/oder - verteilungsschicht (15) insbesondere zwischen dem Topsheet (2) und dem Absorptionskörper (4) angeordnet ist.

12. Inkontinenzartikel nach mindestens einem der vorgenannten Ansprüche **dadurch gekennzeichnet, dass** mindestens 30 Gewichtsprozent, insbesondere mindestens 40 Gewichtsprozent, weiter insbesondere mindestens 50 Gewichtsprozent, weiter insbesondere mindestens 60 Gewichtsprozent, weiter insbesondere mindestens 70 Gewichtsprozent, weiter insbesondere mindestens 80 Gewichtsprozent, weiter insbesondere mindestens 90 Gewichtsprozent, weiter insbesondere 100 Gewichtsprozent des pH-Regulationsmittels (7) zwischen der Flüssigkeitsaufnahme- und/oder -verteilungsschicht (15) und dem Backsheet (3) angeordnet ist.

13. Inkontinenzartikel nach mindestens einem der vorgenannten Ansprüche **dadurch gekennzeichnet, dass** das pH-Regulationsmittel (7) zwischen der Flüssigkeitsaufnahme- und/oder -verteilungsschicht (15) und dem Absorptionskörper (4) angeordnet ist.

14. Inkontinenzartikel nach mindestens einem der vorgenannten Ansprüche **dadurch gekennzeichnet, dass** der Absorptionskörper (4) mindestens einen in der Längsrichtung (11) orientierten Kanal (16), insbesondere genau einen in der Längsrichtung (11) orientierten Kanal (16) aufweist, und wobei der mindestens eine Kanal (16) oder der genau eine Kanal (16) insbesondere symmetrisch, weiter insbesondere spiegelsymmetrisch mit der Längsmittelachse (17) des Absorptionskörpers (4) als Symmetrieachse ausgebildet ist.

15. Inkontinenzartikel nach Anspruch 14 **dadurch gekennzeichnet, dass** der mindestens eine Kanal (16) oder der genau eine Kanal (16) in der ersten Saugkörperlage (5) angeordnet ist und insbesondere eine sich durch die erste Saugkörperlage (5) in Dickenrichtung (21) zumindest teilweise, insbesondere vollständig hindurcherstreckende Ausnehmung umfasst oder daraus gebildet ist.

16. Inkontinenzartikel nach mindestens einem der vorgenannten Ansprüche 14 oder 15 **dadurch gekennzeichnet, dass** der mindestens eine Kanal (16), insbesondere der genau eine Kanal (16), zumindest teilweise, insbesondere vollständig innerhalb des zentralen Bereichs (12) angeordnet ist.

17. Inkontinenzartikel nach mindestens einem der vorgenannten Ansprüche 14 bis 16 **dadurch gekennzeichnet, dass** der Kanal (16) kein pH-Regulationsmittel aufweist.

18. Inkontinenzartikel nach mindestens einem der vorgenannten Ansprüche 14 bis 17 **dadurch gekennzeichnet, dass** die jeweilige Längserstreckung (22,23) des ersten Bereichs (13) und/oder des zweiten Bereichs (14) zumindest gleich groß, insbesondere größer ist als eine Längserstreckung (28) des mindestens einen Kanals (16), derart dass ein Verhältnis der jeweiligen Längserstreckung (22,23) des ersten Bereichs (13) und/oder des zweiten Bereichs (14) zu der Längserstreckung (28) des mindestens einen Kanals (16) einen Wert von mindestens 1,0, insbesondere mindestens 1,1, weiter insbesondere mindestens 1,3 und/oder einen Wert von höchstens 4,0, insbesondere höchstens 3,5, weiter insbesondere höchstens 3,0, weiter insbesondere höchstens 2,7 aufweist.

19. Inkontinenzartikel nach mindestens einem der vorgenannten Ansprüche **dadurch gekennzeichnet, dass** das pH-Regulationsmittel (7) mindestens eine Komponente ausgewählt aus der Gruppe Mononatriumcitrat, Dinatriumcitrat, Trinatriumcitrat aufweist, insbesondere dass das pH-Regulationsmittel (7) aus Mononatriumcitrat besteht, oder aus Dinatriumcitrat besteht, oder aus einer Kombination von Mononatriumcitrat und Dinatriumcitrat besteht.
